(19) Europäisches Patentamt
European Patent Office
Office européen des brevets

(11) Numéro de publication : **0 327 455 B1**

(12) # FASCICULE DE BREVET EUROPEEN

(45) Date de publication du fascicule du brevet : **16.09.92 Bulletin 92/38**

(21) Numéro de dépôt : **89400282.3**

(22) Date de dépôt : **01.02.89**

(51) Int. Cl.$^5$ : **C07C 43/23,** C07C 43/225, C07C 43/178, C07C 205/35, C07C 217/48, C07C 255/37, A61K 31/085, A61K 31/275, A61K 31/13

(54) **Ethers alkyliques ou benzyliques du phénol, leurs procédés de préparation et leur application en thérapeutique.**

(30) Priorité : **05.02.88 FR 8801373**

(43) Date de publication de la demande : **09.08.89 Bulletin 89/32**

(45) Mention de la délivrance du brevet : **16.09.92 Bulletin 92/38**

(84) Etats contractants désignés : **AT BE CH DE ES GB GR IT LI LU NL SE**

(56) Documents cités :
**EP-A- 0 100 033**
**WO-A-84/00106**
**FR-A- 2 528 046**
**LU-A- 65 114**

(73) Titulaire : **DELALANDE S.A.**
**32, rue Henri Regnault**
**F-92400 Courbevoie (FR)**

(72) Inventeur : **Schoffs, Alain-René**
**242, rue de la Croix-Nivert**
**F-75015 Paris (FR)**
Inventeur : **Langlois, Michel**
**4, place César Franck**
**F-78530 Buc (FR)**
Inventeur : **Jeanpetit, Christian**
**19, allée Henri Sellier**
**F-92800 Puteaux (FR)**
Inventeur : **Masson, Maryse**
**45, rue de Trévise**
**F-75009 Paris (FR)**

(74) Mandataire : **Kedinger, Jean-Paul et al**
**c/o Cabinet Malemont 42, avenue du Président Wilson**
**F-75116 Paris (FR)**

EP 0 327 455 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne certains éthers alkyliques ou benzyliques du phénol, faisant preuve d'une activité d'inhibition de la monoamine oxydase et en particulier une activité d'inhibition sélective de la monoamine oxydase de type B.

Par WO-A-8 400 106, LU-A-65 114, EP-A-O 100 033, FR-A-2 528 046, Journal of Organic Chemistry, vol. 50, No 17, p.3125-3132 (1985) et C.A. 93, 1980, 132227j, on connaît déjà des éthers du phénol.

Les éthers selon l'invention répondent plus précisément à la formule :

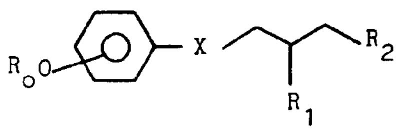

(Io)

dans laquelle :

– $R_o$ représente un groupe alkyle en $C_3$-$C_6$ ou un groupe benzyle de formule :

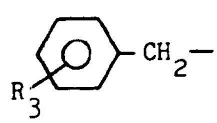

où $R_3$ = H, halogène, alkyle en $C_1$-$C_4$, alkoxy en $C_1$-$C_4$, $CF_3$, $NO_2$ ou CN ; et

– $R_2$ représente :

. un groupe alkoxy en $C_1$-$C_4$, auquel cas le couple (X, $R_1$) = (O, OH), (O, alkoxy en $C_1$-$C_4$), (CHOH, H), (O, H), ($CH_2$, H) ou (CO, H) ;

. un groupe OH, auquel cas le couple (X, $R_1$) = (O, OH), (O, alkoxy en $C_1$-$C_4$), (O, H) ou ($CH_2$, H), avec la réserve que lorsque (X, $R_1$) = (O, OH), Ro est différent de alkyle en $C_3$-$C_6$ et de benzyle ; ou

. un groupe CN ou NH-alkyle en $C_1$-$C_4$, auquel cas le couple (X, $R_1$) = (O, H) ou ($CH_2$, H).

A l'exception des composés pour lesquels l'ensemble ($R_2$, X, $R_1$) prend la signification (OH, O, H) et $R_oO$ représente le groupe p-(Cl 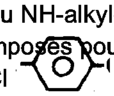 $CH_2O$) ou O-benzyloxy et des composés pour lesquels l'ensemble (X, $R_1$, RO) prend la signification (O, H, p-benzyloxy) et $R_2$ = $NHCH_3$ ou NH-CH$(CH_3)_2$, ces éthers sont tous nouveaux et ces éthers nouveaux répondent à la formule :

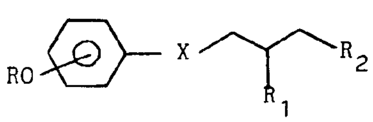

(I)

dans laquelle :

– R représente un groupe alkyle en $C_3$-$C_6$ ou un groupe benzyle de formule :

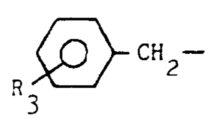

où $R_3$ = H, halogène, alkyle en $C_1$-$C_4$, alkoxy en $C_1$-$C_4$, $CF_3$, $NO_2$ ou CN ; et

– $R_2$ représente :

. un groupe alkoxy en $C_1$-$C_4$, auquel cas le couple (X, $R_1$) = (O, OH), (O, alkoxy en $C_1$-$C_4$), (CHOH, H), (O, H), ($CH_2$, H) ou (CO, H) ;

. un groupe OH, auquel cas le couple (X, $R_1$) = (O, OH), (O, alkoxy en $C_1$-$C_4$), (O, H) ou ($CH_2$, H), avec les réserves que :

* lorsque (X, $R_1$) = (O, OH), R est différent de alkyle en $C_3$-$C_6$ et de benzyle, et

* lorsque (X, $R_1$) = (O, H), RO est différent de para -(Cl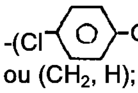$CH_2O$) et de o-benzyloxy;

. un groupe CN, auquel cas le couple (X, $R_1$) = (O, H) ou ($CH_2$, H); ou

. un groupe NH-alkyle en $C_1$-$C_4$, auquel cas le couple (X, $R_1$) = (O, H) ou ($CH_2$, H), avec la réserve que lorsque (X, $R_1$)=(O, H) et $R_2$ = $NHCH_3$ ou NH-CH$(CH_3)_2$, RO est différent de p-benzyloxy.

La présente invention a donc en premier lieu pour objet ces nouveaux éthers (I).

On notera que parmi les éthers ($I_0$) et (I), on préfère notamment ceux pour lesquels RO est situé en position para et, parmi ceux-ci, les éthers pour lesquels $R_3$ est situé en position méta.

D'autre part, quand $R_1$ et $R_2$ représentent un groupe alkoxy en $C_1$-$C_4$, ils représentent de préférence un groupe méthoxy.

On notera par ailleurs que lorsque $R_1$ est différent d'un atome d'hydrogène, les composés correspondants sont chiraux et de ce fait, existent chacun sous la forme de deux énantiomères ; la présente invention s'étend donc à chacun de ces énantiomères et à leurs mélanges y compris le racémique.

Dans ce qui précède et ce qui suit, l'expression "alkyle en $C_3$-$C_6$" comprend les chaînes hydrocarbonées, ramifiées ou linéaires, possédant de 3 à 6 atomes de carbone et notamment n-propyle, iso-propyle, n-butyle, sec-butyle, iso-butyle, ter-butyle, amyle et hexyle ; le terme "halogène" comprend fluor, chlore, brome et iode ; l'expression "alkoxy en $C_1$-$C_4$" possède la formule -O-alkyle en $C_1$-$C_4$ où "alkyle en $C_1$-$C_4$" comprend les chaînes hydrocarbonées, ramifiées ou linéaires, possédant jusqu'à 4 atomes de carbone et notamment méthyle, éthyle, n-propyle, iso-propyle, n-butyle, sec-butyle, iso-butyle et ter-butyle.

La présente invention s'étend par ailleurs aux procédés de préparation des composés (I), ces procédés étant conformes aux schémas réactionnels 1 à 10 ci-après.

Schéma réactionnel 1 :

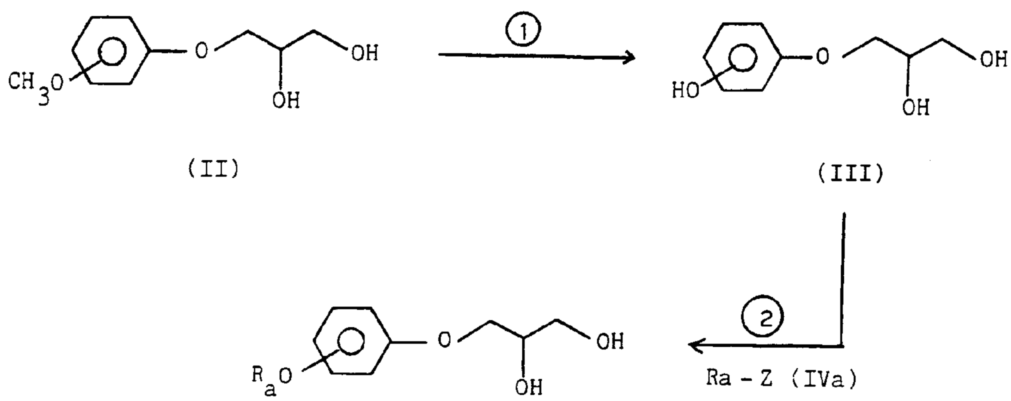


(II)

(III)

(Ia)

Schéma réactionnel 2 :

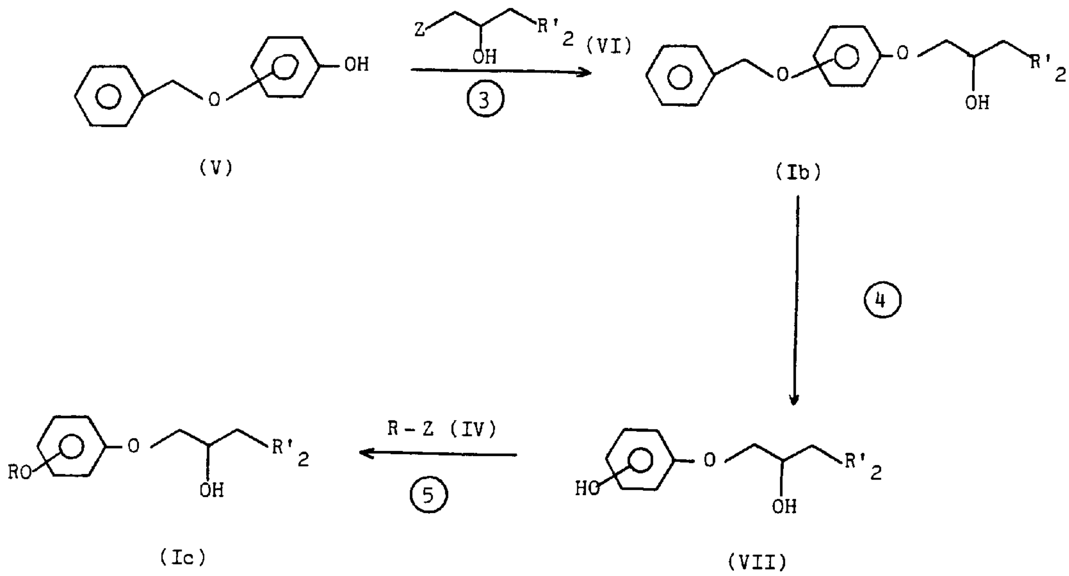


Schéma réactionnel 3 :

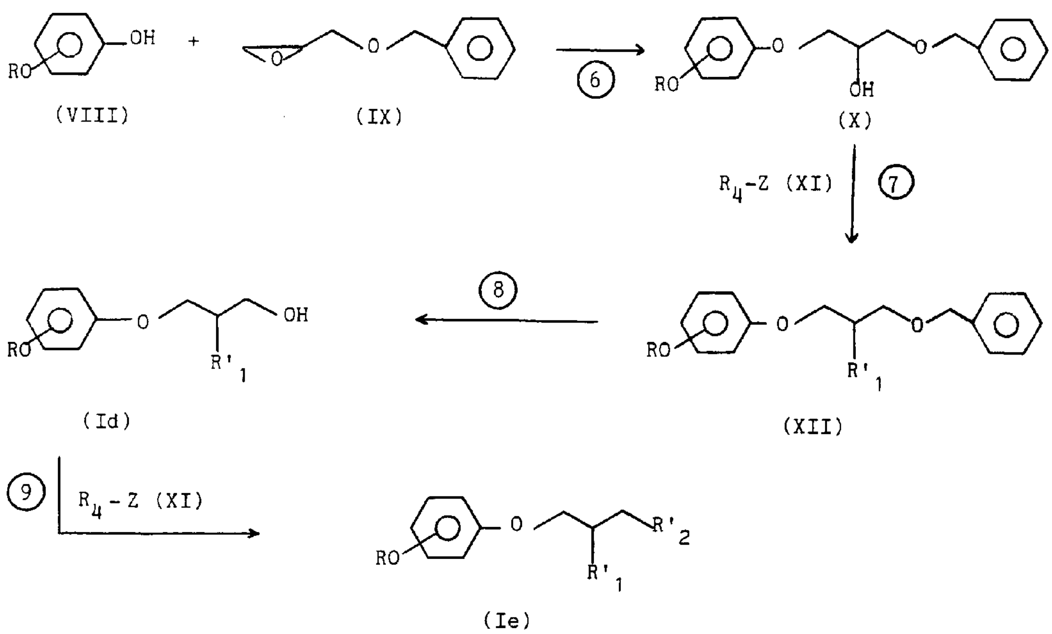

Schéma réactionnel 4 :

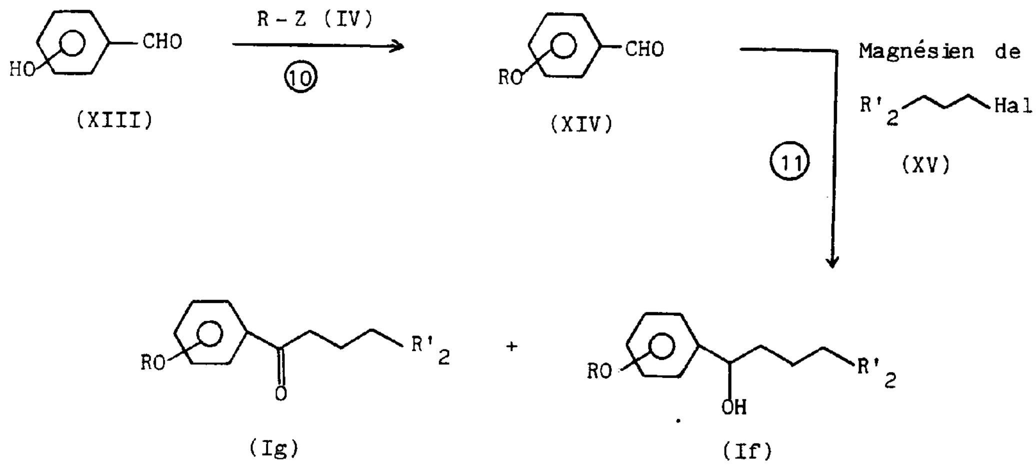


(XIII) (XIV) (XV) (Ig) (If)

Schéma réactionnel 5 :

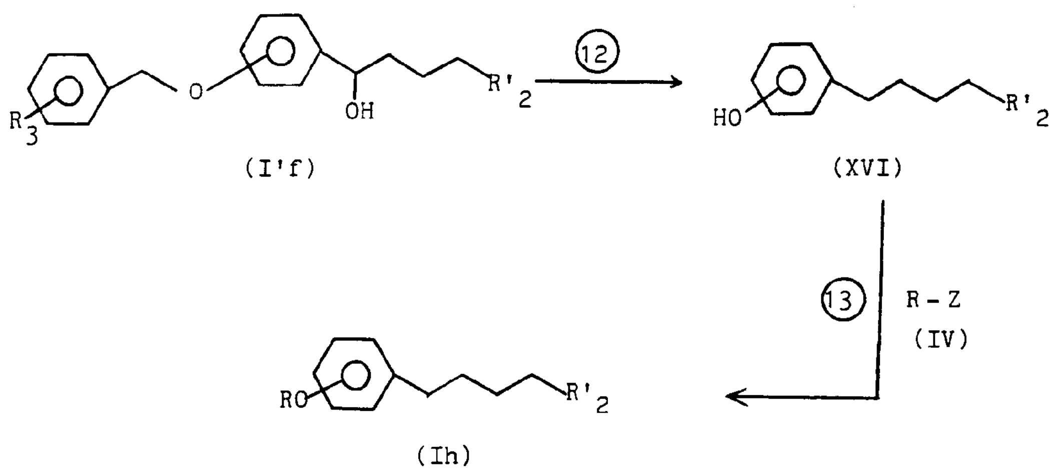


(I'f) (XVI) (Ih)

Schéma réactionnel 6 :

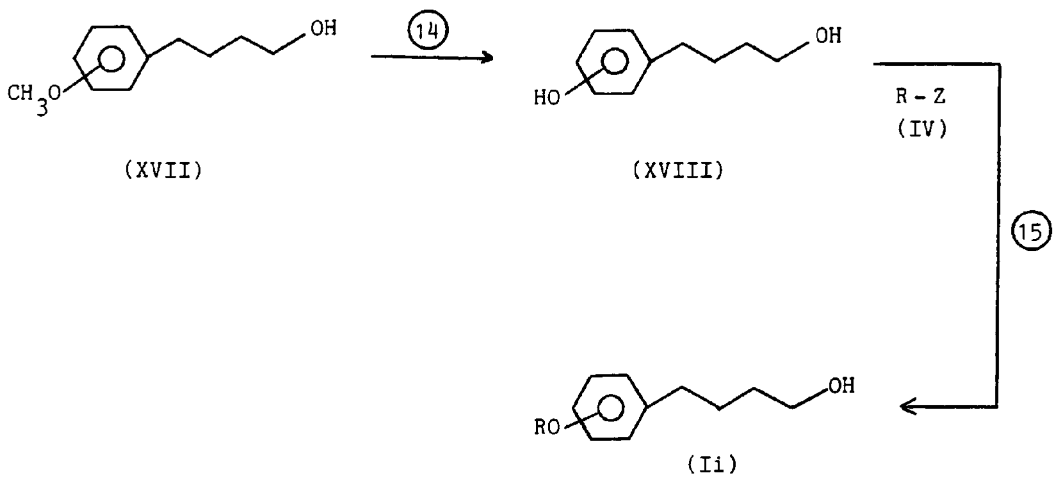


Schéma réactionnel 7 :

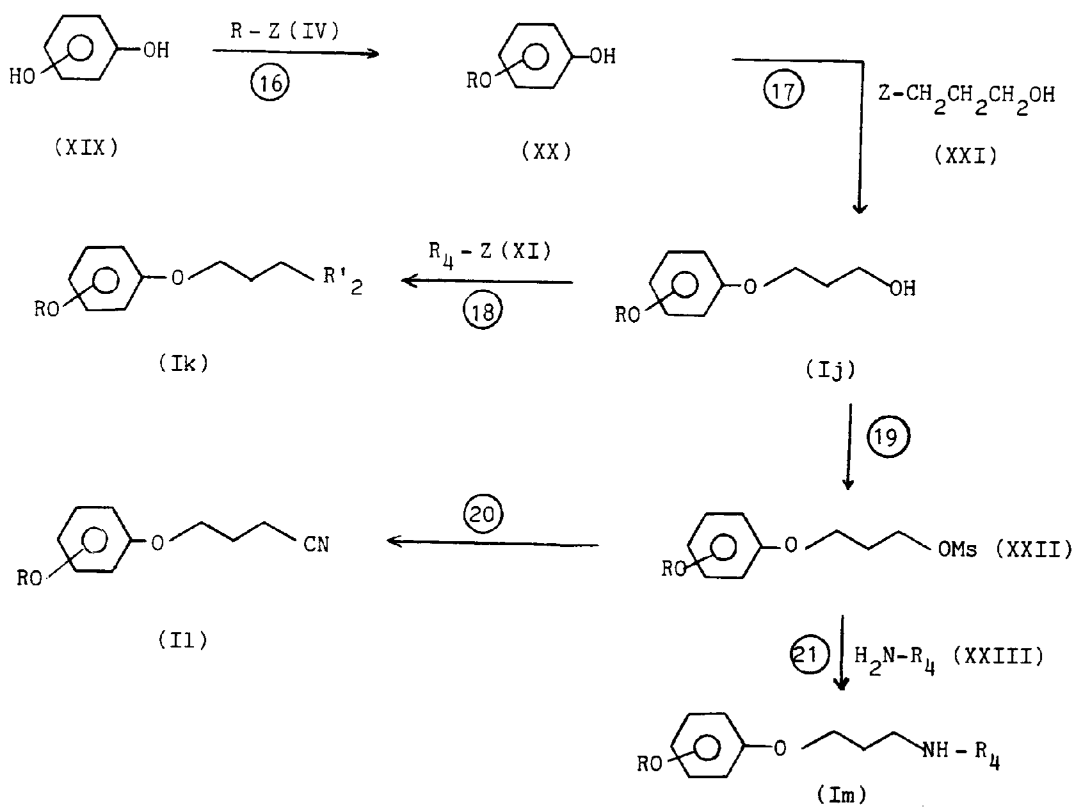

Schéma réactionnel 8 :

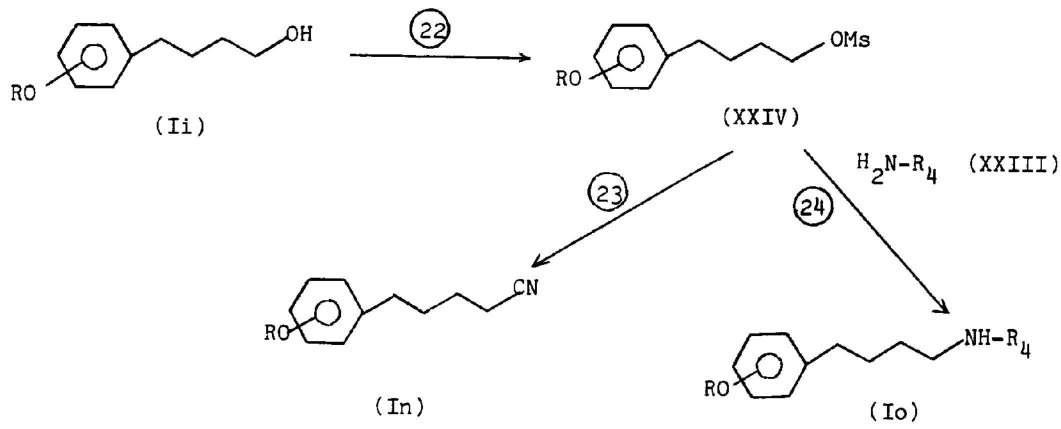


Schéma réactionnel 9 (synthèse stéréospécifique) :

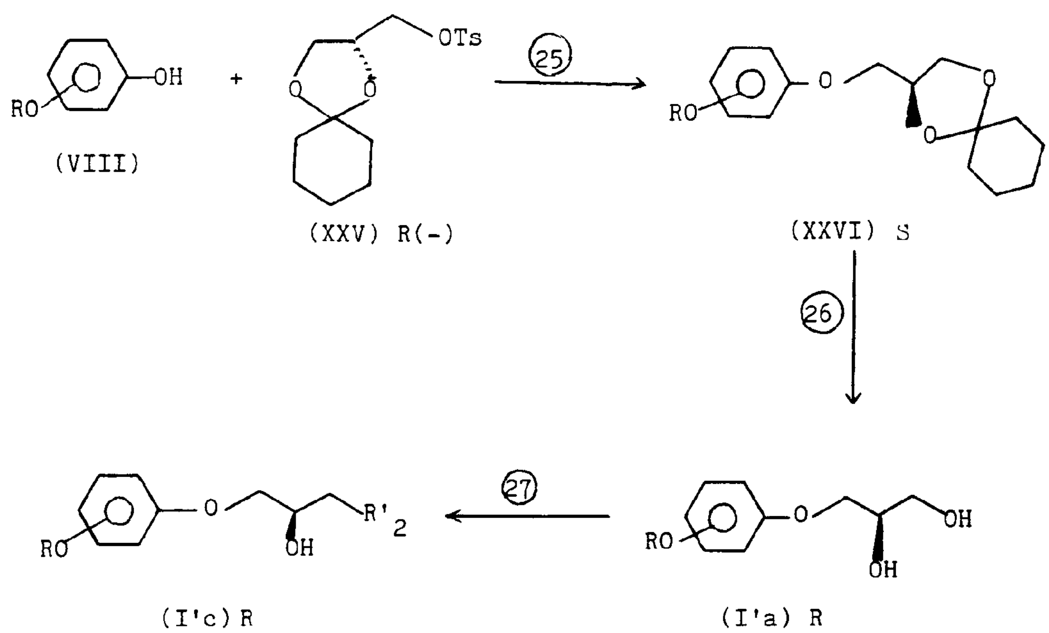

Schéma réactionnel 10 (synthèse stéréospécifique) :

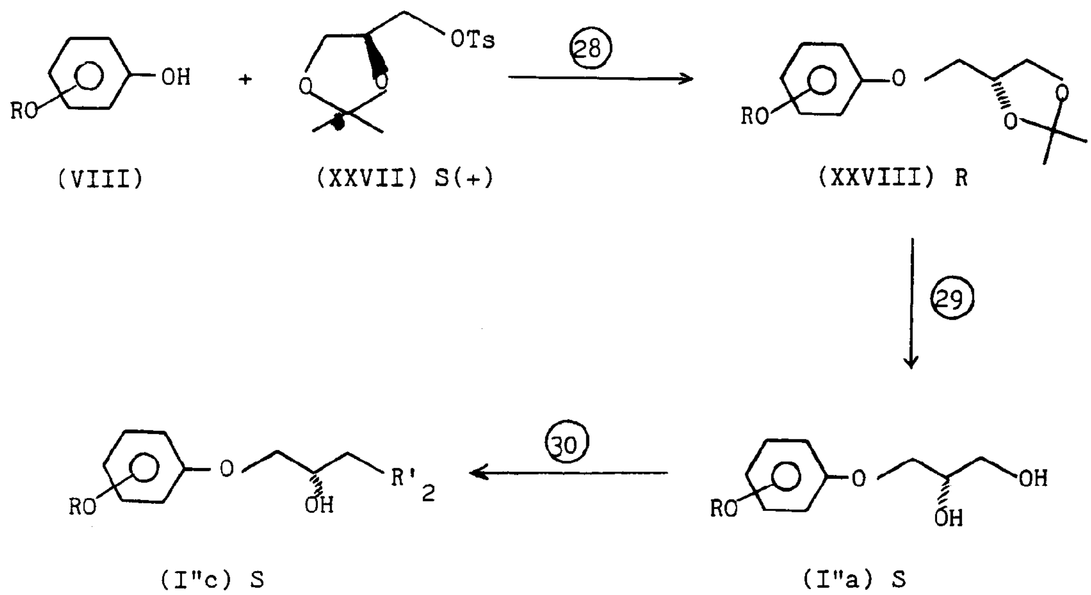


(VIII) (XXVII) S(+) (XXVIII) R

(I"c) S (I"a) S

Les différents symboles apparaissant dans les schémas ci-dessus ont les significations suivantes :

- R : même signification que dans la formule (I),
- Ra : même signification que R dans la formule (I), à l'exception de alkyle en $C_3$-$C_6$ et de benzyle,
- Z : groupe bon partant tel qu'un atome d'halogène,
- R'$_2$ : groupe alkoxy en $C_1$-$C_4$,
- $R_4$ : groupe alkyle en $C_1$-$C_4$,
- R'$_1$ : groupe alkoxy en $C_1$-$C_4$,
- $R_3$ : même signification que dans la formule (I),
- Ms : mésyle,
- Ts : tosyle

(1) (14) : déméthylation à chaud dans HBr à 48 %

(2) (5) (10) (13) (15) (16) (17) (25) (28) : O-alkylation, de préférence en présence d'une base telle que $K_2CO_3$,

(3) : condensation réalisée en présence d'une base telle que KOH et d'un catalyseur de transfert de phase en milieu biphasique

(4) (8) : débenzylation par hydrogénolyse en présence de palladium sur charbon

(6) : condensation en présence d'une base telle que $K_2CO_3$

(7) (9) (18) : O-alkylation en présence d'un hydrure métallique tel que l'hydrure de sodium

(12) : hydrogénation en milieu chlorhydrique et en présence de palladium sur charbon

(19) (22) : action du chlorure de mésyle

(20) (23) : action d'un cyanure de métal alcalin

(26) (29) : hydrolyse acide

(27) (30) : action du chlorure de tosyle suivie de la réaction du tosylate résultant avec l'alcoolate d'un métal alcalin correspondant à l'alcool $R'_2H$.

(11) : action d'un magnésien

(21) (24) : N-alkylation

Il convient de préciser que les tosylates (XXV) et (XXVII) sont préparés selon les procédés classiques bien connus de l'Homme de Métier, à partir des alcools correspondants décrits ou synthétisés selon les modes opératoires de la littérature.

Plus précisément, l'alcool de formule :

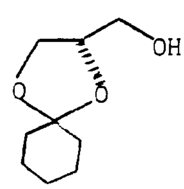

peut être synthétisé par mise en oeuvre du mode opératoire décrit par T. SUGYAMA, H, SUGAWARA, M. WATANABE ET K. YAMASHITA dans Agric. Biol. Chem., 1984, 48, 1841-1844, et l'alcool de formule :

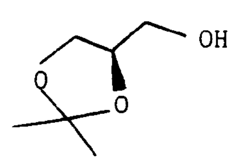

peut être synthétisé par mise en oeuvre des modes opératoires décrits par :

. E. BAER et H.O.L. FISCHER dans J. Amer. Chem. Soc., 1939, 61, 761-765,

. C.M. LOK, J.P. WARD et D.A. VANDORP dans Chem. Phy. Lipids, 1976, 16, 115-122, et

. M.E. JUNG et T.S. SHAW dans J. Amer. Chem. Soc., 1980, 102, 6304-6311.

Par ailleurs, les formes énantiomères des composés (Id) et (Ie) sont synthétisées de manière stéréospécifique selon le schéma réactionnel 3 ci-dessus dans lequel le composé (IX) est soit l'énantiomère

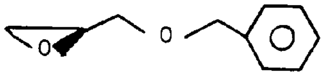 (R), soit l'énantiomère 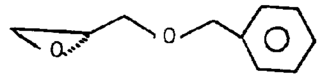 (S),

ces deux énantiomères étant préparés par le protocole décrit par S. TAKANO, M. AKIYAMA et K. OGASAWARA dans Synthesis, 1985, 503-504.

Enfin, les énantiomères des alcools (If) sont obtenus à partir des méthodes classiques de dédoublement rappelées dans "Enantiomers, Racemates and Resolutions" de J. JACQUES, A. COLLET et S.H. WILEN, Wiley-Intersciences, 1981, p. 332-354.

Les préparations suivantes sont données à titre d'exemple pour illustrer l'invention.

Exemple 1 : (hydroxy-4 phénoxy)-3 propanediol-1,2 (±)

[(III) ; numéro de code 340481]

Après addition goutte à goutte de 140 ml de HBr à 48 % sur 30 g (0,151 mole) de (méthoxy-4 phénoxy)-3 propanediol-1,2 (±) à 0° C, le milieu réactionnel est agité pendant 48 heures à 50° C. Après salification progressive par 100 ml $NH_4OH$ 10N et extraction au $CH_2Cl_2$ (pH ~ 1-2), la phase aqueuse est pratiquement évaporée à sec puis épuisée à l'acétate d'éthyle chaud. Le produit brut obtenu recristallisé dans l'acétate d'éthyle laisse 20,5 g d'un solide cristallisé qui correspond au produit attendu (Point de fusion : 132° C).

CCM : gel de silice : $CH_2Cl_2$ - $CH_3OH$ : 90 - 10.

Exemple 2 : (n-propyloxy-4 phénoxy)-3 propanediol-1,2 (±)

[(Ia) ; numéro de code 280327]

A une solution de 2 g (0,011 mole) du produit obtenu à l'exemple 1, dans un mélange $CH_3CN$ - DMF (20 ml - 2 ml), sont ajoutés 1,65 g (0,012 mole) de $K_2CO_3$ puis 4,2 ml (7,4 g ; 0,043 mole) de iodopropane.

Après 16 h à 80° C, le milieu réactionnel est concentré sous pression réduite puis purifié directement par chromatographie flash ($SiO_2$, éluants $CH_2Cl_2$ - $CH_3OH$ : 95,5 - 4,5). On récupère 1,3 g d'un solide blanc qui fond à 95° C et correspond au produit attendu.

CCM : gel de silice : $CH_2Cl_2$ - $CH_3OH$ : 90-10.

Exemple 3 : [(phénylméthyloxy-4) phénoxy]-3 méthoxy-1 propanol-2 (±)

[(Ib) ; numéro de code 280077]

Dans un milieu refroidit par un bain externe à - 10° C, contenant 100 g (0,5 mole) de benzyloxy-4 phénol, 98 g (1,75 mole) de KOH finement broyée et 6 g de catalyseur adogen, sont additionnés goutte à goutte en 1 heure 124 g (1 mole) de chloro-1 méthoxy-3 propanol-2 (±). On porte 16 heures à 80° C, refroidit, extrait à l'acétate d'éthyle (3 x 500 ml), puis lave les phases organiques réunies par NaOH 1N (2 x 500 ml). Après séchage ($MgSO_4$), filtration et évaporation sous pression réduite, il reste un huile brune qui est purifiée par chromatographie flash ($SiO_2$, éluants : heptane - acétate d'éthyle : 65 -35). On isole ainsi 75 g d'un solide blanc qui fond à 63° C et correspond au produit attendu.

CCM : gel de silice, heptane - acétate d'éthyle : 50 - 50.

Exemple 4 : (hydroxy-4 phénoxy)-3 méthoxy-1 propanol-2 (±)

[(VII) ; numéro de code 280078]

Dans une fiole à hydrogéner sont pesés 5,9 g de catalyseur Pd/C à 10% et après addition sous argon de 100 ml d'éthanol sont introduits 59 g (0,20 mole) du produit obtenu à l'exemple 3 en solution dans 400 ml d'éthanol. Après 6 h d'agitation sous atmosphère d'hydrogène la réaction est complète. Après filtration et rinçage du catalyseur suivis d'évaporation, on récupère 36,5 g d'un solide blanc qui fond à 90° C, qui correspond au produit attendu et est utilisé tel quel dans les exemples ci-après.

Exemple 5 : (n-butoxy-4 phénoxy)-3 méthoxy-1 propanol-2 (±)

[(Ic) ; numéro de code 280079]

A une suspension de 10,46 g (0,076 mole) de $K_2CO_3$ dans 50 ml de DMSO contenant 5 g (0,025 mole) du produit obtenu à l'exemple 4, sont ajoutés 5,4 ml (6,91 g ; 0,050 mole) de bromure de butyle. Le mélange réactionnel est agité pendant 16 h à 60° C. Après addition de 20 ml de $H_2O$, les phases organiques provenant de l'extraction par l'acétate d'éthyle (3 x 50 ml) sont lavées par NaOH 1N (5 x 50 ml). Après séchage ($MgSO_4$), filtration et évaporation, l'huile foncée recueillie est purifiée par chromatographie flash ($SiO_2$, éluants : heptane - AcOEt : 65 - 35). L'huile orangée résultante est ensuite distillée au four à boules Büchi. Le produit ainsi obtenu correspond au produit attendu et possède une température d'ébullition sous 0,1 mmHg de 150° C.

CCM : gel de silice $CH_2Cl_2$ - $CH_3OH$ : 95 - 5.

Exemple 6 : [(m-chlorophénylméthyloxy-4) phénoxy]-3 phénylméthyloxy-1 propanol-2 (±)

[(X) ; numéro de code 200211]

Dans un bicol surmonté d'un réfrigérant et placé sous atmosphère d'argon, sont introduits 9 g (0,038 mole) de (chloro-3 benzyloxy-4) phénol, 90 ml de $CH_3CN$, 6,4 g (0,046 mole) de $K_2CO_3$ et 9.5 g (0,058 mole) de phénylméthyloxy méthyloxiranne. Après 16 h d'agitation sous reflux, $K_2CO_3$ est séparé par filtration. Le filtrat est évaporé et repris par l'acétate d'éthyle. La phase organique est lavée par NaOH 1N (3 x 50 ml), séchée ($MgSO_4$), filtrée puis évaporée sous pression réduite. Le produit brut est passé sur chromatographie flash ($SiO_2$, éluants : heptane - acétate d'éthyle : 70 - 30). Le solide blanc ainsi récupéré est recristallisé dans un mélange heptane - acétate d'éthyle :80 - 20. On obtient ainsi 10,4 g d'un solide blanc qui fond à 72° C et correspond au produit attendu.

CCM : gel de silice : heptane - acétate d'éthyle : 70 -30.

Exemple 7 : [(chloro-3 phénylméthyloxy)-4 phénoxy]-1 méthoxy-2 phénylméthyloxy-3 propane (±)

[(XII), numéro de code 200212]

0,66 g (0,022 mole) de NaH lavés deux fois au pentane sont mis en suspension dans 10 ml de THF anhydre. Après refroidissement à 0° C, 8 g (0,020 mole) du produit obtenu à l'exemple 6 en solution dans 70 ml de THF sont additionnés, goutte à goutte, puis 4,3 ml (9,7 g ; 0,060 mole) de $CH_3I$. Après 2 h d'agitation à température ambiante sous atmosphère d'argon, le milieu réactionnel est hydrolysé par addition de 20 ml de $H_2O$ glacée. Après extraction à l'acétate d'éthyle (3 x 80 ml), les phases organiques réunies sont séchées ($MgSO_4$), filtrées et évaporées. On isole 5,6 g d'une huile jaune pâle par chromatographie flash ($SiO_2$, éluants : heptane - acétate d'éthyle : 85 - 15), cette huile correspondant au produit attendu.

Exemple 8 : [(chloro-3 phénylméthyloxy)-4 phénoxy]-3 méthoxy-2 propanol-1 (±)

[(Id) ; numéro de code 200213]

Dans une fiole à hydrogéner à trois tubulures, sont placés 0,4 g de Pd/C à 5 % recouverts de 20 ml de dioxanne. Après purge à l'argon puis à l'hydrogène, 4 g (0,009 mole) du produit à l'exemple 7, en solution dans 20 ml de dioxanne sont introduits. Après 2 h d'agitation à température ambiante, le catalyseur est séparée par filtration et rincé. Le filtrat évaporé sous pression réduite laisse une huile qui cristallise rapidement. Le produit ainsi récupéré est recristallisé dans un mélange heptane - acétate d'éthyle : 80 - 20. On obtient ainsi 2,5 g d'un solide blanc qui fond à 65° C et correspond au produit attendu.

CCM : gel de silice, heptane - acétate d'éthyle : 70 - 30.

Exemple 9 : [(chloro-3 phénylméthyloxy)-4 phénoxy]-1 propanediol-1,2 isopropylidène-1,2 R

[(XXVIII) ; numéro de code 280038]

Dans un tricol surmonté d'un réfrigérant et maintenu sous atmosphère d'argon, sont placés 4,6 g (0,020 mole) de (chloro-3 phénylméthyloxy)-4 phénol, 8,6 g (0,030 mole) de tosylate de glycérol-2,3 isopropylidène

S (+) {$[\alpha]_D^{20}$ + 4,7° (C = 4,0 ; $CH_3OH$)} en solution dans 100 ml de DMF anhydre et 5,5 g (0,040 mole) de $K_2CO_3$. Après 16 h d'agitation à 120° C, les produits minéraux sont séparés par filtration sur Büchner. Le filtrat évaporé est repris par l'acétate d'éthyle et la phase organique lavée par NaOH 1N (3 x 20 ml). Après séchage ($MgSO_4$), filtration et évaporation, l'huile orange résiduelle est purifiée par chromatographie flash ($SiO_2$, éluants : heptane - acétate d'éthyle : 80 - 20) en un solide blanc (3 g) qui correspond au produit attendu.

CCM : gel de silice, heptane - acétate d'éthyle : 60 - 40.

Exemple 10 : [(chloro-3 phénylméthyloxy)-4 phénoxy]-1 propanediol-2,3 S (+)

[(I"a) ; numéro de code 280039]

Le produit obtenu à l'exemple 9 (2,8 g ; 0,008 mole) est mis en solution dans 10 ml de THF. Après 2 h d'agitation en présence de 33 ml HCl 6N, le produit hydrolysé précipite. Il est filtré puis séché sous vide pour donner 2,2 g d'un solide blanc qui correspond au produit attendu.

. $[\alpha]_D^{20}$ = + 4,4° (C = 1 ; $CH_2Cl_2$)

. Point de fusion : 124° C

Exemple 11 : [(chloro-3 phénylméthyloxy)-4 phénoxy]-1 méthoxy-3 propanol-2 S(+)

[(I"c) ; numéro de code 280040]

A une solution de 2,6 ml (2,56 g ; 0,032 mole) de pyridine refroidie à 0° C et contenant 0,8 g (0,004 mole) de chlorure de paratoluène sulfonyle, est introduit, goutte à goutte, 1 g (0,003 mole) du produit obtenu à l'exemple 10 en solution dans 2 ml de pyridine. On abandonne 16 h à température ambiante. Le milieu réactionnel est refroidit à 0° C est on ajoute 4,1 ml d'une solution $CH_3ONa/CH_3OH$ 4,3N (0,018 mole). 16 h d'agitation à température ambiante conduisent à un produit qui est purifié, après évaporation, par chromatographie flash ($SiO_2$, éluants : heptane - acétate d'éthyle : 60 - 40). On isole ainsi 0,5 g d'une solide blanc qui correspond au produit attendu.

. $[\alpha]_D^{20}$ = + 0,6° (C = 1 ; $CH_2Cl_2$)

. Point de fusion : 49° C

Exemple 12 : [(chloro-3 phénylméthyloxy)-4 phénoxy]-1 propanediol-2,3 O.cyclohexylidène S (+)

[(XXVI) ; numéro de code 280041]

A une solution de 9,8 g (0,030 mole) de tosylate de glycérol-2,3 O.cyclohexylidène {$[\alpha]_D^{20}$= - 3,6° (C = 4 ; $CH_3OH$)} et de 4,6 g (0,020 mole) de (chloro-3 phénylméthyloxy)-4 phénol dans 100 ml de DMF anhydre, sont ajoutés 5,5g (0,040 mole) de $K_2CO_3$. Après 16 h d'agitation, les minéraux sont filtrés. Le filtrat évaporé sous pression réduite est repris dans l'acétate d'éthyle (80 ml) et la phase organique est lavée par NaOH 1N (4 x 40 ml), séchée ($MgSO_4$), filtrée et évaporée sous pression réduite. L'huile brune récupérée est purifiée par chromatographie flash ($SiO_2$, éluants heptane - acétate d'éthyle : 85 - 15) pour donner 5,2 g d'une huile jaune pâle correspondant au produit attendu.

$[\alpha]_D^{20}$ = + 8,6° (C = 1 ; $CH_2Cl_2$)

CCM : gel de silice, heptane - acétate d'éthyle : 60 - 40.

Exemple 13 : [(chloro-3 phénylméthyloxy)-4 phénoxy]-3 propanediol-1,2 R (-)

[(I'a) ; numéro de code 280042]

A une solution de 3,4 g (0,009 mole) du produit obtenu à l'exemple 12 dans 15 ml de THF, sont additionnés 34 ml de HCl 6 N. Après 1 h d'agitation à température ambiante, il précipite 2 g d'un solide blanc qui correspond au produit attendu.

. $[\alpha]_D^{20}$ = - 4,8° (C = 1 ; $CH_3OH$)

. Point de fusion : 124° C

CCM : gel de silice, heptane - acétate d'éthyle : 50 - 50.

Exemple 14 : [(chloro-3 phénylméthyloxy)-4 phénoxy]-1 méthoxy-3 propanol-2 R (-)

[(I'c) ; numéro de code 280043]

Dans une solution de 3,1 ml (3,08 g ; 0,039 mole) de pyridine contenant 0,96 g (0,050 mole) de chlorure de tosyle recristallisé maintenue à 0° C par un bain de glace externe, sont ajoutés 1,2 g (0,039 mole) du propanediol-1,2 R (-) obtenu à l'exemple 13, en solution dans 3 ml de pyridine. Après 16 h d'agitation à température ambiante, sous atmosphère d'argon, sont additionnés 4,9 ml de $CH_3ONa$ 4N dans le méthanol à 0° C. Après 16 h à température ambiante, le milieu réactionnel est concentré sous pression réduite. Par purification par chromatographie flash ($SiO_2$, éluants heptane - acétate d'éthyle : 50 - 50), on obtient 0,6 g du produit attendu.

. $[\alpha]_D^{20}$ = - 1° (C = 1 ; $CH_2Cl_2$)

. Pont de fusion : 51° C

CCM : gel de silice, heptane - acétate d'éthyle : 30 - 70.

Exemple 15 : [(chloro-3 phénylméthyloxy)-4] benzaldéhyde

[(XIV) ; numéro de code 280118]

Dans un tricol de 1 l sont introduits 50 g (0,41 mole) de 4-hydroxybenzaldéhyde, 57 ml (72,4 g ; 0,45 mole) de (chloro-3 phényl)-1 chlorométhane, 67,7 g (0,49 mole) de $K_2CO_3$, 400 ml de $CH_3CN$, 40 ml de DMF et 6 g (0,04 mole) de KI. Après 1 h de reflux avec forte agitation, les minéraux sont filtrés. Le filtrat concentré sous pression réduite est repris par 1,5 l d'eau. Le précipité formé est filtré, essoré à l'eau, puis recristallisé dans 200 ml d'éthanol. Après filtration, lavage au pentane, on récupère 90 g d'un solide jaune pâle (F < 60° C) qui correspond au produit attendu.

CCM : gel de silice, heptane - acétate d'éthyle : 70 - 30.

Exemple 16 : [(chloro-3 phénylméthyloxy)-4 phényl]-1 méthoxy-4 butanol-1 (±)

[(If) ; numéro de code 280190] et butanone-1 (±)

[(Ig) ; numéro de code 280189]

A une solution de magnésien préparé à partir de 9,5 g (0,039 mole) de Mg et 8,5 g (0,042 mole) de iodo-1 méthoxy-3 propane, est ajouté goutte à goutte une solution de 9,3 g (0,039 mole) du produit de l'exemple 15 dans 50 ml d'éther anhydre. Après addition, le milieu réactionnel est porté 30 mn à reflux puis hydrolysé par 100 ml d'une solution aqueuse de $NH_4Cl$ 1N. Après décantation, la phase organique est séchée ($MgSO_4$), filtrée et évaporée. Par purification chromatographie flash (éluants : heptane - acétate d'éthyle : 60 - 40), deux produits sont isolés purs :

– le produit le moins polaire étant la butanone-1 attendue, 1 g, F < 60° C
  CCM : gel de silice, heptane - acétate d'éthyle : 50 - 50,
– le produit le plus polaire étant le butanol-1 attendu, 6 g F < 60° C
  CCM : gel de silice, heptane - acétate d'éthyle : 50 - 50.

Exemple 17 : (hydroxy-4 phényl)-1 méthoxy-4 butane

[(XVI) ; numéro de code 280191]

Dans une fiole à hydrogéner, sont placés 3 g (0,01 mole) du butanol -1 (If) obtenu à l'exemple 16, dissous dans 30 ml d'éthanol chlorhydrique 6N et 0,6 g de catalyseur Pd/C 10 % humide à 50 %. Après 4 h d'agitation à température ambiante sous atmosphère d'hydrogène, la réaction est complète. Le catalyseur est séparé par filtration. Après essorage, lavage à l'éthanol et évaporation sous pression réduite, il reste 1,7 g d'une huile limpide qui correspond au produit attendu.

CCM : gel de silice, heptane - acétate d'éthyle : 40 - 60.

Exemple 18 : [(cyano-3 phénylméthyloxy-4) phényl]-1 méthoxy-4 butane

[(Ih) ; numéro de code 280192]

Après 1 h de chauffage à 50° C du milieu réactionnel contenant 2,1 g (0,01 mole) de (cyano-3 phényl)-1 bromométhane, 1,7 g (0,01 mole) du produit obtenu à l'exemple 17 et 3,9 g (0,028 mole) de $K_2CO_3$, sous agitation , la réaction d'alkylation est complète. Les minéraux sont séparés par filtration, la phase organique concentrée est étendue par 100 ml d'eau. Après extraction à l'éther isopropylique, décantation, séchage ($Na_2SO_4$), filtration et évaporation sous pression réduite, on isole une huile qui est distillée au four à boules Büchi (Eb = 210-220° C/1 mmHg) (1,8 g).

CCM : gel de silice-$CH_2Cl_2$.

Exemple 19 : [(chloro-3 phénylméthyloxy)-4 phénol

[(XX) ; numéro de code 200208]

A une suspension de 90,4 g (0,65 mole) de $K_2CO_3$ et 7 g de KI dans 700 ml de $CH_3CN$ contenant 72 g (0,65 mole) de p-hydroquinone, sont ajoutés goutte à goutte 82,9 ml (105,3 g ; 0,65 mole) de métachlorophényl-bromométhane. Après 2 h d'agitation sous reflux, les minéraux sont séparés par filtration et après essorage, le filtrat est évaporé sous pression réduite. Le solide obtenu est repris par 500 ml d'acétate d'éthyle. La phase organique est lavée par NaOH 1 N (3 x 200 ml), séchée ($MgSO_4$), filtrée et évaporée. Le produit brut obtenu est purifié par chromatographie flash ($SiO_2$, éluants $CH_2Cl_2$ - $CH_3OH$ : 98 - 2) en un solide orange (53,3 g) qui fond à 119° C et correspond au produit attendu.

CCM : gel de silice : heptane - acétate d'éthyle : 70 - 30.

Exemple 20 : [(chloro-3 phénylméthyloxy)-4 phényloxy]-3 propanol-1

[(Ij) ; numéro de code 200215]

A une solution de 6,37 g (0,046 mole) de $K_2CO_3$ dans 90 ml de $CH_3CN$ contenant 9 g (0,038 mole) du produit (numéro de code 200208) sont ajoutés goutte à goutte 3,57 ml (4 g ; 0,042 mole) de chloro-3 propanol-1. Après 20 h d'agitation sous reflux, les minéraux sont séparés par filtration et essorés. Le filtrat, après évaporation, est repris par 100 ml d'acétate d'éthyle, la phase organique lavée par NaOH 1N (3 x 50 ml), séchée ($MgSO_4$), filtrée et évaporée sous pression réduite. Le produit brut est purifié par chromatographie flash ($SiO_2$, éluants: heptane - acétate d'éthyle : 50 - 50) en un solide jaune pâle (6 g) fondant à 106°C et correspondant au produit attendu.

CCM : gel de silice, heptane - acétate d'éthyle : 70 - 30.

Exemple 21 : [(chloro-3 phénylméthyloxy)-4 phénoxy]-3 méthoxy-1 propane

[(Ik ) ; numéro de code 200216]

A 0,18 g (0,006 mole) de NaH à 80 % lavé deux fois au pentane et recouvert par 5 ml de THF anhydre sont ajoutés, goutte à goutte, en maintenant la température à 0°C, 10 ml d'une solution de THF anhydre contenant 1,5 g (0,005 mole) du produit obtenu à l'exemple 20. Après 5 mn, on ajoute 1,1 ml (2,5 g : 0,015 mole) de $CH_3I$. Après 4 h d'agitation à température ambiante, la réaction est complète. 5 ml d'eau sont ajoutés et on fait suivre par une extraction à l'acétate d'éthyle (3 x 20 ml). Le traitement usuel est suivi par chromatographie flash ($SiO_2$, éluants : heptane - acétate d'éthyle: 85 - 15) pour obtenir 1,5 g d'une solide blanc (F < 50° C) qui correspond au produit attendu.

CCM gel de silice : heptane - acétate d'éthyle : 70 - 30.

Exemple 22 : [(chloro-3 phénylméthyloxy)-4 phényloxy]-3 mésyloxy-1 propane

[(XXII) ; numéro de code 200217]

Dans 20 ml de $CH_2Cl_2$ sont introduits successivement 2 g (0,007 mole) du produit obtenu à l'exemple 20, 0,64 ml (0,94 g ; 0,008 mole) de chlorure de mésyle, puis à température inférieure à - 5° C, 1,14 ml (0,83 g ; 0,008 mole) de $NEt_3$. Après 3 h d'agitation en laissant la température s'élever, la réaction est complète. Le milieu

est lavé par 2 x 10 ml $H_2O$, et la phase organique séchée ($MgSO_4$) filtrée et évaporée sous pression réduite pour donner 2,4 g d'une huile qui cristallise dans l'éther en un solide crème (1,9g) qui fond à une température inférieure à 50° C et correspond au produit attendu.

Exemple 23 : [(chloro-3 phénylméthyloxy)-4 phényloxy]-3 cyano-1 propane

[(Il) ; numéro de code 200218]

Dans 5 ml de DMSO sont introduits, à 0° C, 0,5 g (0,001 mole) du mésylate de l'exemple 22 et 0,2 g (0,004 mole) de NaCN. Après 24 h d'agitation à température ambiante, le milieu réactionnel est étendu par 2 ml d'eau (échauffement et apparition d'un précipité). Les phases organiques réunies (3 x 5 ml d'acétate d'éthyle) sont séchées ($MgSO_4$), filtrées et évaporées. Le solide jaune pâle (0,35 g) obtenu est recristallisé dans un mélange heptane - acétate d'éthyle : 80 - 20). On isole ainsi 0,3 g d'un solide blanc (Point de fusion : 71° C) qui correspond au produit attendu.

CCM : gel de silice : heptane - acétate d'éthyle : 50 -50.

Exemple 24 : [(chloro-3 phénylméthyloxy)-4 phényloxy]-3 N-méthylamino-1 propane

[(Im) ; numéro de code 200219]

Dans une fiole sont placés 0,8 g (0,002 mole) du mésylate obtenu à l'exemple 22 en solution dans un mélange EtOH - $CH_2Cl_2$ : 50 - 50 (8 ml) et 0,87 ml (0,67 g ; 0,022 mole) de N-méthylamine, refroidis. L'agitation est maintenue 16 h dans ce récipient hermétiquement fermé. Après évaporation partielle, le milieu réactionnel est repris par 30 ml d'acétate d'éthyle et 10 ml d'eau et traité par NaOH 1N (3 x 10 ml).

La phase organique est séchée ($MgSO_4$) filtrée et évaporée sous pression réduite. On obtient ainsi une huile orangée correspondant au produit attendu et qui cristallise lentement. Par traitement dans l'éther à l'aide de 1,4 ml d'éthanol chlorhydrique 2N, on obtient 0,6 g du chlorhydrate correspondant (Point de fusion : 173° C).

CCM : gel de silice : $CH_2Cl_2$ - $CH_3OH$ : 95-5.

Les tableaux I à III ci-après rassemblent les propriétés chimiques d'un certain nombre de composés selon l'invention.

TABLEAU I

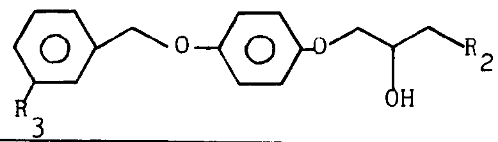

(I)

| Numéro de Code | $R_3$ | $R_2$ | Configuration absolue | Formule brute | Poids moléculaire | ANALYSE ELEMENTAIRE % | C | H | N | Point de fusion (°C) | $[\alpha]_D^{20}$ |
|---|---|---|---|---|---|---|---|---|---|---|---|
| | | | | | | | | | | | |
| 340482 | F | OH | (±) | $C_{16}H_{17}FO_4$ | 292,296 | Cal. | 65,74 | 5,86 | - | 114 | |
| | | | | | | Tr. | 65,88 | 5,97 | - | | |
| 340494 | Cl | OH | (±) | $C_{16}H_{17}ClO_4$ | 308,753 | Cal. | 62,24 | 5,55 | - | 120 | |
| | | | | | | Tr. | 62,00 | 5,68 | - | | |
| 340495 | I | OH | (±) | $C_{16}H_{17}IO_4$ | 400,200 | Cal. | 48,02 | 4,28 | - | 135 | |
| | | | | | | Tr. | 47,98 | 4,30 | - | | |

TABLEAU I (suite)

| Numéro de Code | $R_3$ | $R_2$ | Configuration absolue | Formule brute | Poids moléculaire | ANALYSE ELEMENTAIRE % | C | H | N | Point de fusion (°C) | $[\alpha]_D^{20}$ |
|---|---|---|---|---|---|---|---|---|---|---|---|
| 280331 | Br | OH | (±) | $C_{16}H_{17}BrO_4$ | 353,206 | Cal. | 54,40 | 4,85 | - | 126 | |
| | | | | | | Tr. | 54,00 | 4,89 | - | | |
| 280329 | $CF_3$ | OH | (±) | $C_{17}H_{17}F_3O_4$ | 342,306 | Cal. | 59,65 | 5,01 | - | 78 | |
| | | | | | | Tr. | 59,36 | 4,92 | - | | |
| 280328 | $CH_3O$ | OH | (±) | $C_{17}H_{20}O_5$ | 304,331 | Cal. | 67,09 | 6,62 | - | 74 | |
| | | | | | | Tr. | 67,04 | 6,50 | - | | |
| 280330 | $NO_2$ | OH | (±) | $C_{16}H_{17}NO_6$ | 319,304 | Cal. | 60,18 | 5,37 | 4,39 | 61 | |
| | | | | | | Tr. | 60,09 | 5,30 | 4,31 | | |

TABLEAU I (suite)

| Numéro de Code | $R_3$ | $R_2$ | Configuration absolue | Formule brute | Poids moléculaire | ANALYSE ELEMENTAIRE | | | | Point de fusion (°C) | $[\alpha]_D^{20}$ |
|---|---|---|---|---|---|---|---|---|---|---|---|
| | | | | | | % | C | H | N | | |
| 280415 | CN | OH | (±) | $C_{17}H_{17}NO_4$ | 299,314 | Cal. | 68,21 | 5,73 | 4,68 | 87 | |
| | | | | | | Tr. | 67,66 | 5,88 | 4,48 | | |
| 280039 | Cl | OH | S(+) | $C_{16}H_{17}ClO_4$ | 308,753 | Cal. | - | - | - | 124 | + 4,4°* |
| | | | | | | Tr. | - | - | - | | |
| 280042 | Cl | OH | R(-) | $C_{16}H_{17}ClO_4$ | 308,753 | Cal. | - | - | - | 124 | - 4,8°** |
| | | | | | | Tr. | - | - | - | | |
| 200209 | Cl | $OCH_3$ | (±) | $C_{17}H_{19}ClO_4$ | 322,779 | Cal. | 63,25 | 5,93 | - | 50 | |
| | | | | | | Tr. | 63,47 | 6,15 | - | | |

* (C = 1 ; $CH_2Cl_2$)
** (C = 1 ; $CH_3OH$)

TABLEAU I (suite)

| Numéro de Code | $R_3$ | $R_2$ | Configuration absolue | Formule brute | Poids moléculaire | ANALYSE ELEMENTAIRE % | C | H | N | Point de fusion (°C) | $[\alpha]_D^{20}$ |
|---|---|---|---|---|---|---|---|---|---|---|---|
| 280040 | Cl | $OCH_3$ | S(+) | $C_{17}H_{19}ClO_4$ | 322,779 | Cal. | 63,25 | 5,93 | - | 49 | + 0,6°* |
| | | | | | | Tr. | 63,51 | 6,06 | - | | |
| 280043 | Cl | $OCH_3$ | R(-) | $C_{17}H_{19}ClO_4$ | 322,779 | Cal. | 63,25 | 5,93 | - | 51 | - 1°* |
| | | | | | | Tr. | 63,45 | 5,71 | - | | |
| 280417 | Br | $OCH_3$ | (±) | $C_{17}H_{19}BrO_4$ | 367,231 | Cal. | 55,60 | 5,22 | - | 64 | |
| | | | | | | Tr. | 55,35 | 5,38 | - | | |
| 280418 | I | $OCH_3$ | (±) | $C_{17}H_{19}IO_4$ | 414,226 | Cal. | 49,29 | 4,62 | - | 75 | |
| | | | | | | Tr. | 49,59 | 4,63 | - | | |

* (C = 1 ; $CH_2Cl_2$)

TABLEAU I (suite)

| Numéro de Code | $R_3$ | $R_2$ | Configuration absolue | Formule brute | Poids moléculaire | ANALYSE ELEMENTAIRE | | | | Point de fusion (°C) | $[\alpha]_D^{20}$ |
|---|---|---|---|---|---|---|---|---|---|---|---|
| | | | | | | % | C | H | N | | |
| 280416 | CN | $OCH_3$ | (±) | $C_{18}H_{19}NO_4$ | 313,340 | Cal. | 68,99 | 6,11 | 4,47 | <60 | |
| | | | | | | Tr. | 68,04 | 6,04 | 4,52 | | |
| 280077 | H | $OCH_3$ | (±) | $C_{17}H_{20}O_4$ | 313,340 | Cal. | - | - | - | 63 | |
| | | | | | | Tr. | - | - | - | | |
| | | | | | | Cal. | | | | | |
| | | | | | | Tr. | | | | | |
| | | | | | | Cal. | | | | | |
| | | | | | | Tr. | | | | | |

TABLEAU II

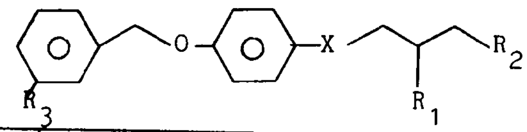

(I)

| Numéro de Code | $R_3$ | X | $R_1$ | $R_2$ | Configuration absolue | Formule brute | Poids moléculaire | ANALYSE ELEMENTAIRE % | C | H | N | Point de fusion (°C) |
|---|---|---|---|---|---|---|---|---|---|---|---|---|
| 200123 | Cl | $CH_2$ | H | OH | - | $C_{17}H_{19}ClO_2$ | 290,779 | Cal. | 70,21 | 6,59 | - | 52 |
| | | | | | | | | Tr. | 69,57 | 6,76 | - | |
| 200124 | Cl | $CH_2$ | H | $OCH_3$ | - | $C_{18}H_{21}ClO_2$ | 304,805 | Cal. | 70,92 | 6,94 | - | Huile |
| | | | | | | | | Tr. | 71,13 | 7,13 | - | |
| 200215 | Cl | O | H | OH | - | $C_{16}H_{17}ClO_3$ | 292,753 | Cal. | 65,64 | 5,85 | - | 106 |
| | | | | | | | | Tr. | 65,65 | 5,77 | - | |
| 200216 | Cl | O | H | $OCH_3$ | - | $C_{17}H_{19}ClO_3$ | 306,779 | Cal. | 66,55 | 6,24 | - | $>50$ |
| | | | | | | | | Tr. | 66,26 | 6,34 | - | |

TABLEAU II (suite)

| Numéro de Code | $R_3$ | X | $R_1$ | $R_2$ | Configuration absolue | Formule brute | Poids moléculaire | ANALYSE ELEMENTAIRE % | C | H | N | Point de fusion (°C) |
|---|---|---|---|---|---|---|---|---|---|---|---|---|
| 200219 | Cl | O | H | $NHCH_3$ | - | $C_{17}H_{20}ClNO_2,HCl$ | 342,260 | Cal. | 59,65 | 6,18 | 4,09 | 173 |
| | | | | | | | | Tr. | 59,49 | 6,23 | 4,23 | |
| 200218 | Cl | O | H | CN | - | $C_{17}H_{16}ClNO_2$ | 301,763 | Cal. | 67,66 | 5,34 | 4,64 | 71 |
| | | | | | | | | Tr. | 67,62 | 5,39 | 4,70 | |
| 200213 | Cl | O | $OCH_3$ | OH | (±) | $C_{17}H_{19}ClO_4$ | 322,779 | Cal. | 63,25 | 5,93 | - | 65 |
| | | | | | | | | Tr. | 63,27 | 5,93 | - | |
| 200210 | Cl | O | $OCH_3$ | $OCH_3$ | (±) | $C_{18}H_{21}ClO_4$ | 336,805 | Cal. | 64,19 | 6,28 | - | Huile |
| | | | | | | | | Tr. | 63,99 | 6,53 | - | |

TABLEAU II (suite)

| Numéro de Code | $R_3$ | X | $R_1$ | $R_2$ | Configuration absolue | Formule brute | Poids moléculaire | ANALYSE ELEMENTAIRE % | C | H | N | Point de fusion (°C) |
|---|---|---|---|---|---|---|---|---|---|---|---|---|
| 280189 | Cl | CO | H | $OCH_3$ | - | $C_{18}H_{19}ClO_3$ | 318,789 | Cal. | 67,81 | 6,01 | - | <60 |
| | | | | | | | | Tr. | 67,76 | 6,13 | - | |
| 280190 | Cl | CHOH | H | $OCH_3$ | (±) | $C_{18}H_{21}ClO_3$ | 320,805 | Cal. | 67,39 | 6,60 | - | <60 |
| | | | | | | | | Tr. | 67,28 | 6,68 | - | |
| 280192 | CN | $CH_2$ | H | $OCH_3$ | - | $C_{19}H_{21}NO_2$ | 295,366 | Cal. | 77,26 | 7,17 | 4,74 | * |
| | | | | | | | | Tr. | 76,64 | 7,22 | 4,29 | |

* : Eb = 210-220°C/1mmHg

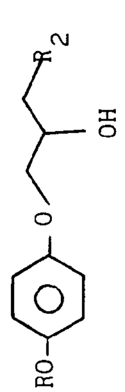

(I)

TABLEAU III

| Numéro de Code | RO | $R_2$ | Configuration absolue | Formule brute | Poids moléculaire | ANALYSE ELEMENTAIRE % | C | H | N | Point de fusion (°C) |
|---|---|---|---|---|---|---|---|---|---|---|
| | | | | | | | | | | |
| | | | | | | | | | | |
| 280079 | $C_4H_9O$ | $OCH_3$ | (±) | $C_{14}H_{22}O_4$ | 254,316 | Cal. | 66,11 | 8,72 | - | Huile |
| | | | | | | Tr. | 65,82 | 8,99 | - | |
| 280080 | $C_5H_{11}O$ | $OCH_3$ | (±) | $C_{15}H_{24}O_4$ | 268,342 | Cal. | 67,13 | 9,02 | - | Huile |
| | | | | | | Tr. | 67,20 | 9,15 | - | |

Les composés ($I_o$) précédemment définis ont été étudiés chez l'animal de laboratoire et ont montré une activité inhibitrice de la monoamine oxydase et notamment une activité inhibitrice sélective de la monoamine oxydase de type B.

Cette activité a été mise en évidence par un test permettant la mesure in-vitro de l'effet inhibiteur sur les deux formes A et B de la monoamine oxydase de cerveau de rat, selon un protocole adapté de J. Pharm. Pharmacol., 1983, 35, 161-165.

Les résultats obtenus avec quelques composés ($I_o$) sont répertoriés dans le tableau IV ci-après.

TABLEAU IV

Constantes d'inhibition $K_{IA}$ et $K_{IB}$ des formes A et B de la monoamine oxydase (homogénat de cerveau total de rat mâle Sprague-Dawley à 1 g de tissu pour 16 ml de tampon phosphate pH 7,4)

| Code du composé testé | INHIBITION IN VITRO DE LA MONOAMINE OXYDASE | | | | | |
|---|---|---|---|---|---|---|
| | sans préincubation | | | avec préincubation (pendant 20 min.) | | |
| | $K_{IA}$ | $K_{IB}$ | $K_{IA}/K_{IB}$ | $K_{IA}$ | $K_{IB}$ | $K_{IA}/K_{IB}$ |
| 280040 | - | - | - | 1648 | 9 | 183 |
| 200123 | > 1724 | 232 | > 7 | - | - | - |
| 200213 | > 5172 | 543 | > 9,5 | - | - | - |
| 200210 | > 1724 | 403 | > 4 | > 1724 | 570 | > 3 |
| 280190 | - | - | - | 5431 | 79 | 69 |

Les constantes d'inhibition ont été calculées d'après la formule :

$$K_I = CI_{50}/I + [S]/K_m$$

avec

[S] = 48 µM pour la [$^{14}$C] sérotonine et $K_m$ = 100 µM

[S] = 12 µM pour la [$^{14}$C] phényléthylamine et $K_m$ = 6 µM

substrats sélectifs respectivement des formes A et B de la monoamine oxydase.

Par ailleurs, après administration chez le rongeur des composés ($I_o$) à une dose de 100 mg/kg/p.o., il n'a pas été observé de signes de toxicité après 24 heures.

Les composés ($I_o$) peuvent donc être utilisés pour la préparation de médicaments inhibiteurs de la monoamine oxydase et plus particulièrement de médicaments inhibiteurs sélectifs de la monoamine oxydase de type B, ces médicaments trouvant leur emploi en thérapeutique notamment pour le traitement des troubles neurologiques liés au vieillissement pathologique, des troubles mnésiques, de l'humeur, de la schizophrénie, de la psychasténie ou ralentissement psychique lié au vieillissement, de certaines formes de dépression et de la maladie de Parkinson.

Ces médicaments peuvent être administrés à l'Homme ou à tout animal à sang chaud, sous diverses formes pharmaceutiques bien connues dans la technique considérée, et notamment sous la forme de compositions formulées en vue de l'administration par voie orale, parentérale ou rectale.

Pour l'administration par voie orale, lesdites compositions peuvent prendre la forme de comprimés, dragées ou gélules, préparés par les techniques habituelles utilisant des supports et excipients connus tels que des agents liants, des charges, des lubrifiants et des agents de désintégration ; elles peuvent également prendre la forme de solutions, de sirops ou de suspensions.

Pour l'administration par voie parentérale, les compositions selon l'invention peuvent prendre la forme de solutions, suspensions ou émulsions injectables comprenant un véhicule liquide, huileux ou aqueux, parentéralement acceptable.

Pour l'administration par voie rectale, les compositions peuvent prendre la forme de suppositoires comprenant les bases habituelles pour suppositoires.

La dose à laquelle les principes actifs, c'est-à-dire les composés ($I_0$) peuvent être administrés dépendra notamment de la voie d'administration, du poids corporel du patient et de la puissance thérapeutique des composés mis en oeuvre. Généralement, par voie orale, les doses pourront atteindre 200 mg de principe actif par jour (en une ou plusieurs prises) ; par voie parentérale, les doses pourront atteindre 300 mg de principe actif par jour (en une ou deux injections journalières) ; et par voie rectale, les doses pourront atteindre 300 mg de principe actif par jour (en un ou deux suppositoires).

## Revendications

### Revendications pour les Etats contractants suivants : AT, DE, BE, IT, LU, NL, GB, SE, CH, LI

**1.** Composés de formule :

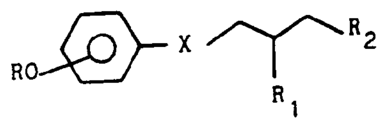

(I)

dans laquelle :

– R représente un groupe alkyle en $C_3$-$C_6$ ou un groupe benzyle de formule :

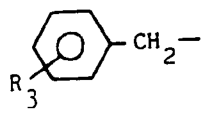

où $R_3$ = H, halogène, alkyle en $C_1$-$C_4$, alkoxy en $C_1$-$C_4$, $CF_3$, $NO_2$ ou CN ; et

– $R_2$ représente :

. un groupe alkoxy en $C_1$-$C_4$, auquel cas le couple (X, $R_1$) = (O, OH), (O, alkoxy en $C_1$-$C_4$), (CHOH, H), (O, H), ($CH_2$, H) ou (CO, H) ;

. un groupe OH, auquel cas le couple (X, $R_1$) = (O, OH), (O, alkoxy en $C_1$-$C_4$), (O, H) ou ($CH_2$, H), avec les réserves que :

* lorsque (X, $R_1$) = (O, OH), R est différent de alkyle en $C_3$-$C_6$ et de benzyle, et

* lorsque (X, $R_1$) = (O, H), RO est différent de para -(Cl 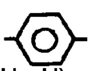 $CH_2O$) et de o-benzyloxy ;

. un groupe CN, auquel cas le couple (X,$R_1$)=(O, H) ou ($CH_2$, H); ou

. un groupe NH-alkyle en $C_1$-$C_4$, auquel cas le couple (X, $R_1$)=(O,H) ou ($CH_2$, H), avec la réserve qe lorsque (X, $R_1$) = (O, H), et $R_2$ = NH-$CH_3$ ou

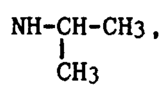

RO est différent de p-benzyloxy.

**2.** Composés selon la revendication 1, pour lesquels RO est situé en position para.

**3.** Composés selon la revendication 2, pour lesquels $R_3$ est situé en position méta.

**4.** Composés selon l'une des revendications précédentes, pour lesquels $R_1$ est un groupe méthoxy.

**5.** Composés selon l'une des revendications 1 à 4, pour lesquels $R_2$ est un groupe méthoxy.

**6.** Composition pharmaceutique ou vétérinaire comprenant (a) au moins un composé de formule :

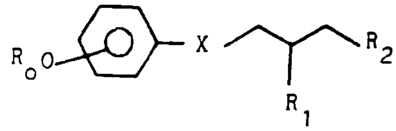

(Io)

dans laquelle :

– $R_o$ représente un groupe alkyle en $C_3$-$C_6$ ou un groupe benzyle de formule :

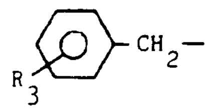

où $R_3$ = H, halogène, alkyle en $C_1$-$C_4$, alkoxy en $C_1$-$C_4$, $CF_3$, $NO_2$ ou CN ; et

– $R_2$ représente :

. un groupe alkoxy en $C_1$-$C_4$, auquel cas le couple (X, $R_1$) = (O, OH), (O, alkoxy en $C_1$-$C_4$), (CHOH, H), (O, H), ($CH_2$, H) ou (CO, H) ;

. un groupe OH, auquel cas le couple (X, $R_1$) = (O, OH), (O, alkoxy en $C_1$-$C_4$), (O, H) ou ($CH_2$, H), avec la réserve que lorsque (X, $R_1$) = (O, OH), $R_o$ est différent de alkyle en $C_3$-$C_6$ et de benzyle ; ou

. un groupe CN ou NH-alkyle en $C_1$-$C_4$, auquel cas le couple (X, $R_1$) = (O, H) ou ($CH_2$, H),

et (b) un support ou véhicule physiologiquement acceptable et approprié pour ce composé.

**7.** Utilisation des composés ($I_o$) définis à la revendication 6 pour la préparation d'un agent inhibiteur sélectif de la monoamine oxydase de type B.

**8.** Procédé de préparation des composés de formule :

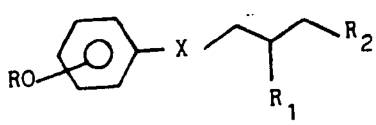

(I)

dans laquelle :

– R représente un groupe alkyle en $C_3$-$C_6$ ou un groupe benzyle de formule :

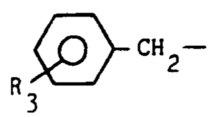

où $R_3$ = H, halogène, alkyle en $C_1$-$C_4$, alkoxy en $C_1$-$C_4$, $CF_3$, $NO_2$ ou CN ; et

– $R_2$ représente :

. un groupe alkoxy en $C_1$-$C_4$, auquel cas le couple (X, $R_1$) = (O, OH), (O, alkoxy en $C_1$-$C_4$), (CHOH, H), (O, H), ($CH_2$, H) ou (CO, H) ;

. un groupe OH, auquel cas le couple (X, $R_1$) = (O, OH), (O, alkoxy en $C_1$-$C_4$), (O, H) ou ($CH_2$, H), avec les réserves que :

* lorsque (X, $R_1$) = (O, OH), R est différent de alkyle en $C_3$-$C_6$ et de benzyle, et

* lorsque (X, $R_1$) = (O, H), RO est différent de para -(Cl 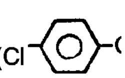 $CH_2O$) et de o-benzyloxy ;

. un groupe CN, auquel cas le couple (X,$R_1$)=(O, H) ou ($CH_2$, H); ou

. un groupe NH-alkyle en $C_1$-$C_4$, auquel cas le couple (X, $R_1$)=(O,H) ou ($CH_2$, H), avec la réserve que lorsque (X, $R_1$) = (O, H), et $R_2$ = NH-$CH_3$ ou

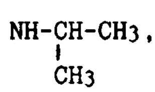

RO est différent de p-benzyloxy,

caractérisé en ce qu'il comprend :

(a) l'alkylation des phénols de formule :

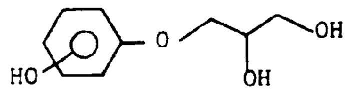

(III)

respectivement par les composés de formule :

Ra -Z (IVa)

où Z est un groupe bon partant et Ra a la même signification que R dans la formule (I), à l'exception de alkyle en $C_3$-$C_6$ et de benzyle, ce qui conduit aux composés de formule :

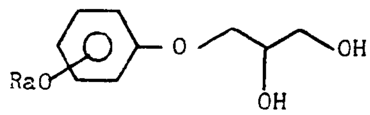

(Ia) ;

(b) la condensation des composés de formule :

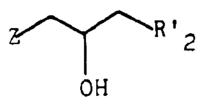

(VI)

où Z est un groupe bon partant et R'$_2$ est un groupe alkoxy en $C_1$-$C_4$, respectivement sur un benzyloxyphénol, en présence d'une base et d'un catalyseur de transfert de phase en milieu biphasique, ce qui conduit aux composés de formule :

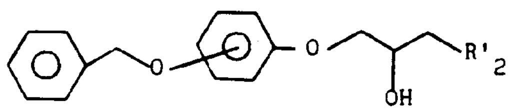

(Ib)

où R'$_2$ a la même signification que dans la formule (VI) ;

(c) l'alkylation des composés de formule :

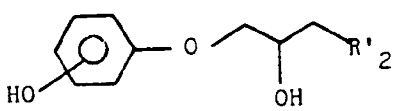

(VII)

où R'$_2$ a la même signification que dans la formule (VI), respectivement par les composés de formule :

R - Z (IV)

où R a la même signification que dans la formule (I) et Z est un groupe bon partant,
ce qui conduit aux composés de formule :

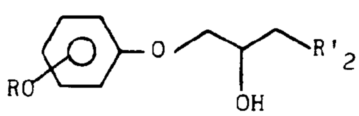

(Ic)

où R a la même signification que dans la formule (I) et R'$_2$ a la même signification que dans la formule (VI) ;

(d) le clivage par hydrogénation des composés de formule :

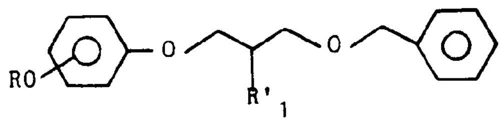

(XII)

sous l'une de ses formes énantiomères ou sous la forme d'un mélange de ces dernières,
où R a la même signification que dans la formule (I) et $R'_1$ représente un groupe alkoxy en $C_1$-$C_4$, en présence de palladium sur charbon, ce qui conduit aux composés de formule :

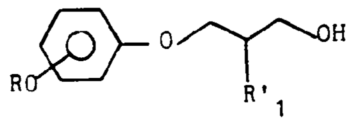

(Id)

où R et $R'_1$ ont les mêmes significations que dans la formule (XII) ;
(e) l'alkylation en présence d'un hydrure métallique des composés (Id) respectivement par un composé de formule :

$$R_4 - Z \qquad (XI)$$

où Z est un groupe bon partant et $R_4$ représente un groupe alkyle en $C_1$-$C_4$, ce qui conduit aux composé de formule :

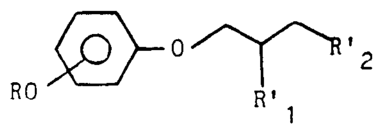

(Ie)

où R et $R'_1$ ont les mêmes significations que dans la formule (XII) et $R'_2$ = alkoxy en $C_1$-$C_4$ ;
(f) la réaction avec les composés de formule :

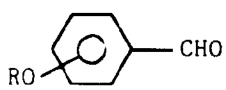

(XIV)

où R a la même signification que dans la formule (I), du magnésien d'un composé de formule :

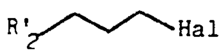

(XV)

où $R'_2$ représente un groupe alkoxy en $C_1$-$C_4$ et Hal un atome d'halogène, ce qui conduit à un mélange des composés de formules

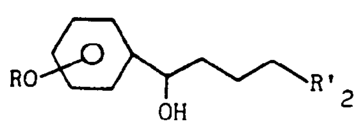

(If)

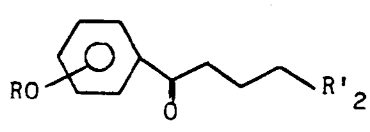

(Ig),

où R a la même signification que dans la formule (I) et $R'_2$ représente un groupe alkoxy en $C_1$-$C_4$, cette alkylation étant suivie de la séparation par chromatographie des composés (If) et (Ig) ;
(g) l'alkylation en présence d'une base, des composés de formule :

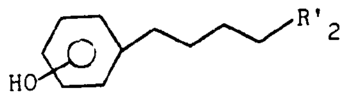

(XVI)

où $R'_2$ représente un groupe alkoxy en $C_1$-$C_4$, respectivement par les composés (IV), ce qui conduit aux composés de formule :

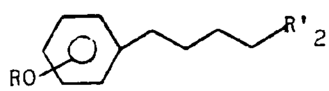

(Ih)

où R et $R'_2$ ont les mêmes significations que dans la formule (If) ;
(h) l'alkylation en présence d'une base des phénols de formule :

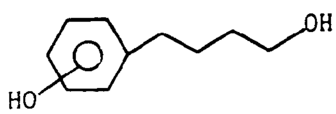

(XVIII)

respectivement par les composés de formule (IV), ce qui conduit aux composés de formule :

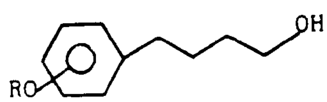

(Ii)

où R a la même signification que dans la formule (I) ;
(i) l'alkylation en présence d'une base des phénols de formule :

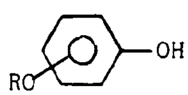

(XX)

où R a la même signification que dans la formule (I), respectivement par les composés de formule :

$$Z - CH_2CH_2CH_2 - OH \qquad (XXI)$$

où Z est un groupe bon partant, ce qui conduit aux composés de formule :

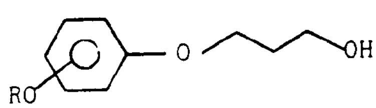

(Ij)

où R a la même signification que dans la formule (I), suivie d'une O-alkylation de ces derniers par les composés de formule ((XI) en présence d'un hydrure métallique, ce qui conduit aux composés de formule :

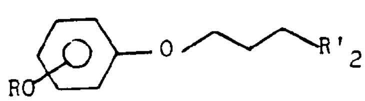

(Ik)

où R a la même signification que dans la formule (I) et $R'_2$ représente un groupe alkoxy en $C_1$-$C_4$ ;
(j) la réaction du cyanure de sodium sur les composés de formule :

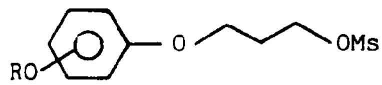 (XXII),

ou

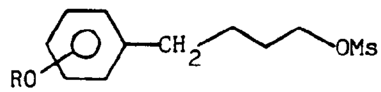 (XXIV)

où R a la même signification que dans la formule (I) et Ms représente un groupe mésyle, ce qui conduit respectivement aux composés de formule :

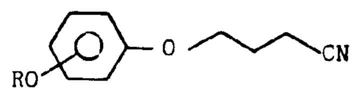 (Il),

ou

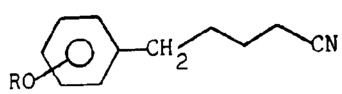 (In)

où R a la même signification que dans la formule (I) ;
(k) la réaction des N-alkylamines de formule :

$H_2N$ - $R_4$ (XXIII)

où $R_4$ représente un groupe alkyle en $C_1$-$C_4$, sur les composés de formule (XXII) ou (XXIV), ce qui conduit aux composés de formule

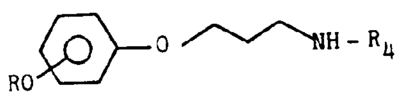 (Im),

ou

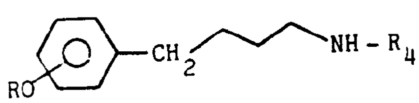 (Io)

où R a la même signification que dans la formule (I) et $R_4$ représente un groupe alkyle en $C_1$-$C_4$ ;
(l) l'hydrolyse acide des composés de formule :

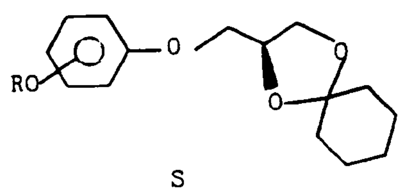 (XXVI),

S

ou

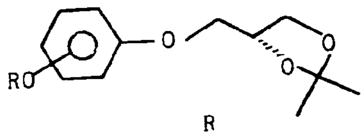
(XXVIII)

R

où R a la même signification que dans la formule (I), ce qui conduit aux composés de formule :

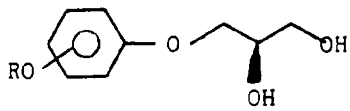
(I'a),

R

ou

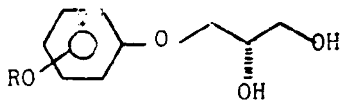
(I"a) ;

S

et

(m) l'action du chlorure de tosyle sur les composés de formule (I'a) ou (I″a), suivie de la réaction des tosylates résultants respectivement avec un alcoolate de métal alcalin correspondant à l'alcool, de formule $R'_2H$ où $R'_2$ représente un groupe alkoxy en $C_1$-$C_4$, ce qui conduit aux composés de formule :

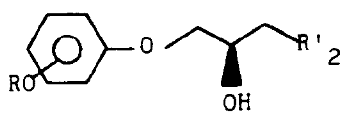
(I'c),

R

ou

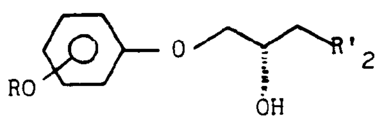
(I"c)

S

**Revendications pour les Etats contractants suivants: ES, GR**

**1.** Procédé de préparation des composés de formule :

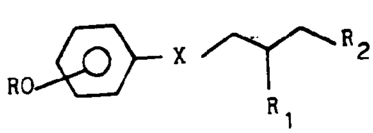
(I)

dans laquelle :

– R représente un groupe alkyle en $C_3$-$C_6$ ou un groupe benzyle de formule :

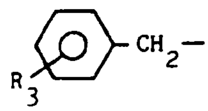

où $R_3$ = H, halogène, alkyle en $C_1$-$C_4$, alkoxy en $C_1$-$C_4$, $CF_3$, $NO_2$ ou CN ; et

– $R_2$ représente :

. un groupe alkoxy en $C_1$-$C_4$, auquel cas le couple (X, $R_1$) = (O, OH), (O, alkoxy en $C_1$-$C_4$), (CHOH, H), (O, H), ($CH_2$, H) ou (CO, H) ;

. un groupe OH, auquel cas le couple (X, $R_1$) = (O, OH), (O, alkoxy en $C_1$-$C_4$), (O, H) ou ($CH_2$, H), avec les réserves que :

. lorsque (X, $R_1$) = (O, OH), R est différent de alkyle en $C_3$-$C_6$ et de benzyle, et

* lorsque (X, $R_1$) = (O, H), RO est différent de para -(Cl 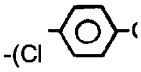 $CH_2O$) et de o-benzyloxy ;

. un groupe CN, auquel cas le couple (X,$R_1$)=(O, H) ou ($CH_2$, H); ou

. un groupe NH-alkyle en $C_1$-$C_4$, auquel cas le couple (X, $R_1$)=(O,H) ou ($CH_2$, H), avec la réserve que lorsque (X, $R_1$) = (O, H), et $R_2$ = NH-$CH_3$ ou

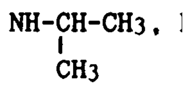

RO est différent de p-benzyloxy,

caractérisé en ce qu'il comprend :

(a) l'alkylation des phénols de formule :

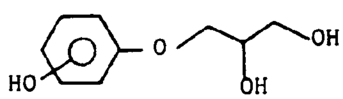 (III)

respectivement par les composés de formule :

Ra - Z (IVa)

où Z est un groupe bon partant et Ra a la même signification que R dans la formule (I), à l'exception de alkyle en $C_3$-$C_6$ et de benzyle, ce qui conduit aux composés de formule :

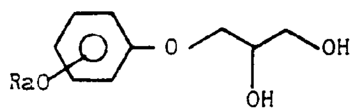 (Ia) ;

(b) la condensation des composés de formule :

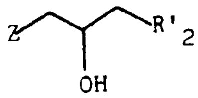 (VI)

où Z est un groupe bon partant et R'$_2$ est un groupe alkoxy en $C_1$-$C_4$, respectivement sur un benzyloxyphénol, en présence d'une base et d'un catalyseur se transfert de phase en milieu biphasique, ce qui conduit aux composés de formule :

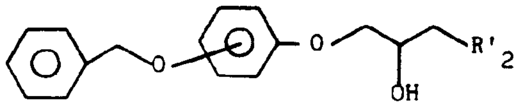

(Ib)

où $R'_2$ a la même signification que dans la formule (VI) ;
(c) l'alkylation des composés de formule :

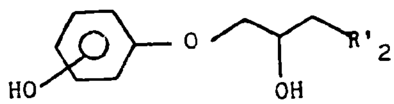

(VII)

où $R'_2$ a la même signification que dans la formule (VI), respectivement par les composés de formule :

R - Z (IV)

où R a la même signification que dans la formule (I) et Z est un groupe bon partant,
ce qui conduit aux composés de formule :

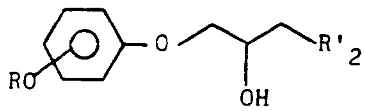

(Ic)

où R a la même signification que dans la formule (I) et $R'_2$ a la même signification que dans la formule (VI) ;
(d) le clivage par hydrogénation des composés de formule :

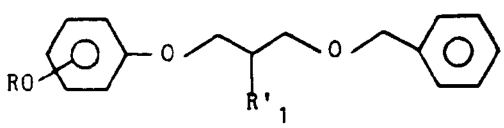

(XII)

sous l'une de ses formes énantiomères ou sous la forme d'un mélange de ces dernières,
où R a la même signification que dans la formule (I) et $R'_1$ représente un groupe alkoxy en $C_1$-$C_4$, en présence de palladium sur charbon, ce qui conduit aux composés de formule :

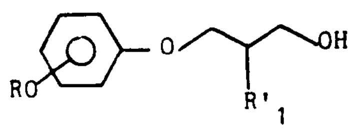

(Id)

où R et $R'_1$ ont les mêmes significations que dans la formule (XII) ;
(e) l'alkylation en présence d'un hydrure métallique des composés (Id) respectivement par un composé de formule :

$R_4$ - Z (XI)

où Z est un groupe partant et $R_4$ représente un groupe alkyle en $C_1$-$C_4$, ce qui conduit aux composé de formule :

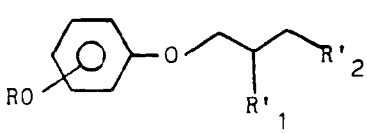

(Ie)

où R et $R'_1$ ont les mêmes significations que dans la formule (XII) et $R'_2$ = alkoxy en $C_1$-$C_4$ ;

(f) la réaction avec les composés de formule :

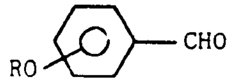

(XIV)

où R a la même signification que dans la formule (I), du magnésien d'un composé de formule :

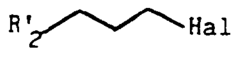

(XV)

où $R'_2$ représente un groupe alkoxy en $C_1$-$C_4$ et Hal un atome d'halogène, ce qui conduit à un mélange des composés de formules

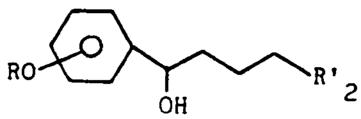

(If)

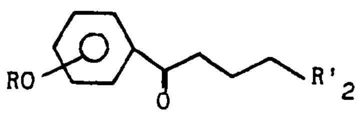

(Ig),

où R a la même signification que dans la formule (I) et $R'_2$ représente un groupe alkoxy en $C_1$-$C_4$, cette alkylation étant suivie de la séparation par chromatographie des composés (If) et (Ig) ;
(g) l'alkylation en présence d'une base, des composés de formule :

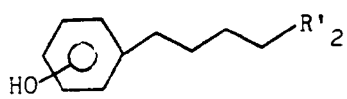

(XVI)

où $R'_2$ représente un groupe alkoxy en $C_1$-$C_4$, respectivement par les composés (IV), ce qui conduit aux composés de formule :

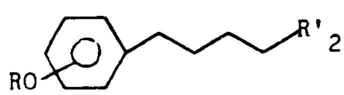

(Ih)

où R et $R'_2$ ont les mêmes significations que dans la formule (If) ;
(h) l'alkylation en présence d'une base des phénols de formule :

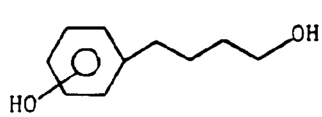

(XVIII)

respectivement par les composés de formule (IV), ce qui conduit aux composés de formule :

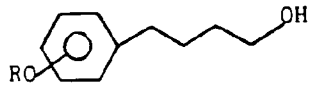

(Ii)

où R a la même signification que dans la formule (I) ;
(i) l'alkylation en présence d'une base des phénols de formule :

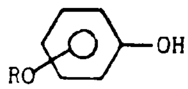

(XX)

où R a la même signification que dans la formule (I), respectivement par les composés de formule :

$$Z - CH_2CH_2CH_2 - OH \qquad (XXI)$$

où Z est un groupe bon partant, ce qui conduit aux composés de formule :

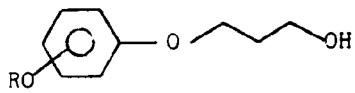

(Ij)

où R a la même signification que dans la formule (I), suivie d'une O-alkylation de ces derniers par les composés de formule (XI) en présence d'un hydrure métallique, ce qui conduit aux composés de formule :

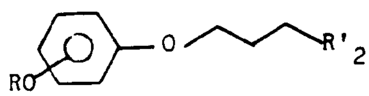

(Ik)

où R a la même signification que dans la formule (I) et $R'_2$ représente un groupe alkoxy en $C_1$-$C_4$ ;
(j) la réaction du cyanure de sodium sur les composés de formule :

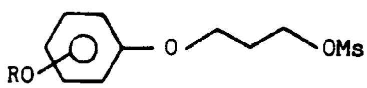

(XXII),

ou

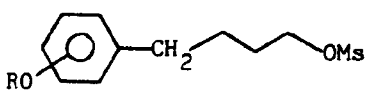

(XXIV)

où R a la même signification que dans la formule (I) et Ms représente un groupe mésyle, ce qui conduit respectivement aux composés de formule :

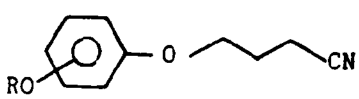

(Il),

ou

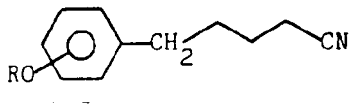

(In)

où R a la même signification que dans la formule (I) ;

(k) la réaction des N-alkylamines de formule :

$H_2N - R_4$ (XXIII)

où $R_4$ représente un groupe alkyle en $C_1$-$C_4$, sur les composés de formule (XXII) ou (XXIV), ce qui conduit aux composés de formule

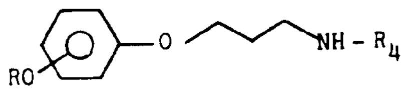

(Im),

ou

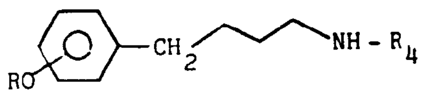

(Io)

où R a la même signification que dans la formule (I) et $R_4$ représente un groupe alkyle en $C_1$-$C_4$ ;

(l) l'hydrolyse acide des composés de formule :

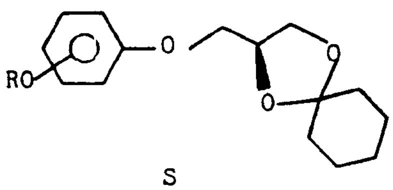

S

(XXVI),

ou

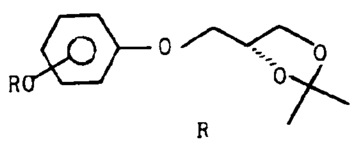

R

(XXVIII)

où R a la même signification que dans la formule (I), ce qui conduit aux composés de formule :

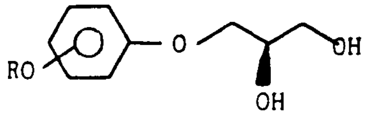

R

(I'a),

ou

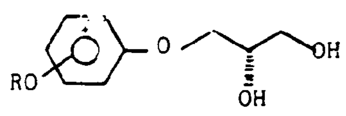 (I"a) ;

S

et

(m) l'action du chlorure de tosyle sur les composés de formule (I'a) ou (I"a), suivie de la réaction des tosylates résultants respectivement avec un alcoolate de métal alcalin correspondant à l'alcool de formule $R'_2H$ où $R'_2$ représente un groupe alkoxy en $C_1$-$C_4$, ce qui conduit aux composés de formule :

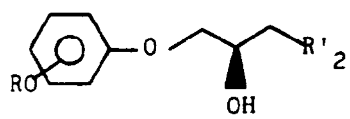 (I'c),

R

ou

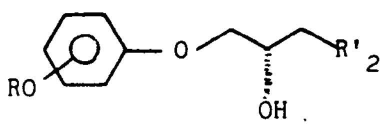 (I"c)

S

2. Procédé selon la revendication 1, caractérisé en ce que l'alkylation (a), (c), (g), (h) ou (i) est effectuée en présence de $K_2CO_3$.

**Patentansprüche**

**Patentansprüche für folgende Vertragsstaaten : AT, DE, BE, IT, LU, NL, GB, SE, CH, LI**

1. Verbindungen der Formel

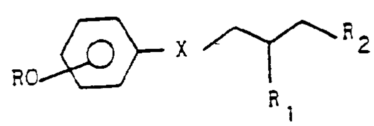 (I)

in welcher

– R eine $C_3$-$C_6$ Alkyl-Gruppe oder eine Benzyl-Gruppe der Formel

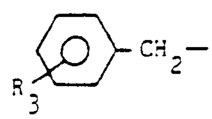

bedeutet, worin $R_3$ = H, Halogen, $C_1$-$C_4$ Alkyl, $C_1$-$C_4$ Alkoxy, $CF_3$, $NO_2$ oder CN; und

– $R_2$

. eine $C_1$-$C_4$ Alkoxy-Gruppe bedeutet, in welchem Fall die Paarung (X, $R_1$) = (O, OH), (O, $C_1$-$C_4$ Alkoxy), (CHOH, H), (O, H), ($CH_2$, H) oder (CO, H);

. eine OH-Gruppe bedeutet, in welchem Fall die Paarung (X, $R_1$) = (O, OH), (O, $C_1$-$C_4$ Alkoxy), (O, H) oder ($CH_2$, H ), mit den Maßgaben, daß:

* wenn (X, $R_1$) = (O, OH), R von $C_3$-$C_6$ Alkyl und Benzyl verschieden ist, und

* wenn (X, $R_1$) = (O, H), RO von para -( Cl 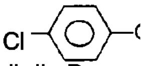 $CH_2O$ ) und o-Benzyloxy verschieden ist;

. eine CN-Gruppe bedeutet, in welchem Fall die Paarung (X, $R_1$) = (O, H) oder ($CH_2$, H); oder

. eine NH-$C_1$-$C_4$ Alkyl-Gruppe bedeutet, in welchem Fall die Paarung (X, $R_1$) = (O, H) oder ($CH_2$, H), mit der Maßgabe, daß, wenn (X, $R_1$) = (O, H) und $R_2$ = NH-$CH_3$ oder

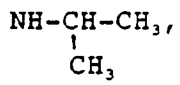

RO von p-Benzyloxy verschieden ist.

2. Verbindungen gemäß Anspruch 1, bei welchen sich RO in der para-Stellung befindet.

3. Verbindungen gemäß Anspruch 2, bei welchen sich $R_3$ in meta-Stellung befindet.

4. Verbindungen gemäß einem der vorstehenden Ansprüche, bei welchen $R_1$ eine Methoxy-Gruppe ist.

5. Verbindungen gemäß einem der Ansprüche 1 bis 4, bei welchen $R_2$ eine Methoxy-Gruppe ist.

6. Pharmazeutische oder veterinärmedizinische Zusammensetzung, enthaltend (a) wenigstens eine Verbindung der Formel

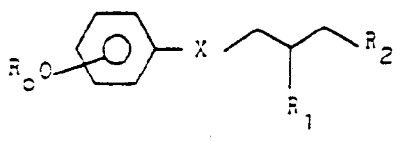

(Io)

in welcher

– Ro eine $C_3$-$C_6$ Alkyl-Gruppe oder eine Benzyl-Gruppe der Formel

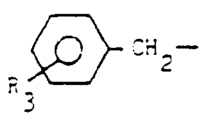

bedeutet, worin $R_3$ = H, Halogen, $C_1$-$C_4$ Alkyl, $C_1$-$C_4$ Alkoxy, $CF_3$, $NO_2$ oder CN; und

– $R_2$

. eine $C_1$-$C_4$ Alkoxy-Gruppe bedeutet, in welchem Fall die Paarung (X, $R_1$) = (O, OH), (O, $C_1$-$C_4$ Alkoxy), (CHOH, H), (O, H), ($CH_2$, H) oder (CO, H);

. eine OH-Gruppe bedeutet, in welchem Fall die Paarung (X, $R_1$) = (O, OH), (O, $C_1$-$C_4$ Alkoxy), (O, H) oder ($CH_2$, H), mit der Maßgabe, daß wenn (X, $R_1$) = (O, OH), Ro von $C_3$-$C_6$ Alkyl und Benzyl verschieden ist, oder

. eine CN- oder NH-$C_1$-$C_4$ Alkyl-Gruppe bedeutet, in welchem Fall die Paarung (X, $R_1$) = (O, H) oder ($CH_2$, H),

und (b) einem physiologisch annehmbaren und für diese Verbindung geeigneten Träger.

7. Verwendung von wie in Anspruch 6 definierten Verbindungen (Io) zur Herstellung eines selektiven Inhibierungsmittels für Monoaminoxidase des Typs B.

8. Verfahren zur Herstellung von Verbindungen der Formel

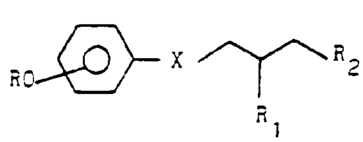

(I)

in welcher
– R eine $C_3$-$C_6$ Alkyl-Gruppe öder eine Benzyl-Gruppe der Formel

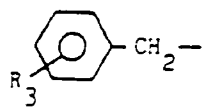

bedeutet, worin $R_3$ = H, Halogen, $C_1$-$C_4$ Alkyl, $C_1$-$C_4$ Alkoxy, $CF_3$, $NO_2$ oder CN; und
– $R_2$
. eine $C_1$-$C_4$ Alkoxy-Gruppe bedeutet, in welchem Fall die Paarung (X, $R_1$) = (O, OH), (O, $C_1$-$C_4$ Alkoxy), (CHOH, H), (O, H), ($CH_2$, H) oder (CO, H);
. eine OH-Gruppe bedeutet, in welchem Fall die Paarung (X, $R_1$) = (O, OH), (O, $C_1$-$C_4$ Alkoxy), (O, H) oder ($CH_2$, H), mit den Maßgaben, daß:
* wenn (X, $R_1$) = (O, OH), R von $C_3$-$C_6$ Alkyl und Benzyl verschieden ist, und
* wenn (X, $R_1$) = (O, H), RO von para -(Cl 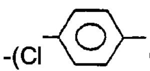 $CH_2O$ ) und o-Benzyloxy verschieden ist;
. eine CN-Gruppe bedeutet, in welchem Fall die Paarung (X, $R_1$) = (O, H) oder ($CH_2$, H); oder
. eine NH-$C_1$-$C_4$ Alkyl-Gruppe bedeutet, in welchem Fall die Paarung (X, $R_1$) = (O, H) oder ($CH_2$, H), mit der Maßgabe, daß, wenn (X, $R_1$) = (O, H) und $R_2$ = NH-$CH_3$ oder

NH-CH-$CH_3$,
$CH_3$

RO von p-Benzyloxy verschieden ist,
dadurch gekennzeichnet, däß es
(a) die Alkylierung von Phenolen der Formel

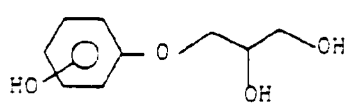
(III)

durch die Verbindungen der Formel

Ra - Z (IVa)

worin Z eine Abgangsgruppe ist und Ra dieselbe Bedeutung wie R in der Formel (I) mit Ausnahme von $C_3$-$C_6$ Alkyl und Benzyl hat, was zu Verbindungen der Formel

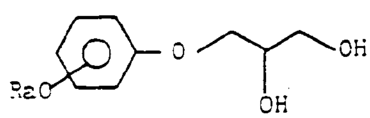
(Ia)

führt,
(b) der Kondensation von Verbindungen der Formel

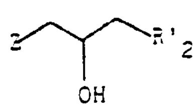
(VI)

worin Z eine Abgangsgruppe ist und $R'_2$ eine $C_1$-$C_4$ Alkoxy-Gruppe beziehungsweise ein Benzyloxyphenol ist, in Gegenwart einer Base und eines Phasentransferkatalysators in einem Zweiphasensystem, was zu Verbindungen der Formel

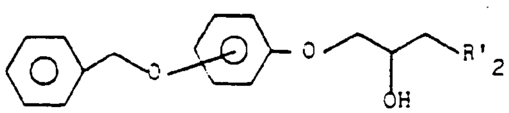

(Ib)

führt, worin $R'_2$ dieselbe Bedeutung wie in Formel (VI) hat;

(c) die Alkylierung von Verbindungen der Formel

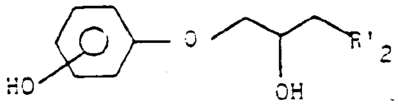

(VII)

worin $R'_2$ dieselbe Bedeutung wie in Formel (VI) hat, durch die Verbindungen der Formel

R - Z (IV)

worin R dieselbe Bedeutung wie in Formel (I) hat und Z eine Abgangsgruppe ist, was zu Verbindungen der Formel

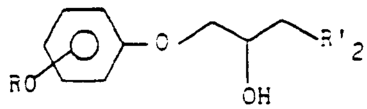

(Ic)

führt, worin R dieselbe Bedeutung wie in Formel (I) hat und $R'_2$ dieselbe Bedeutung wie in Formel (VI) hat;

(d) die Spaltung von Verbindungen der Formel

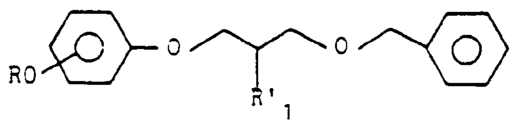

(XII)

durch Hydrierung in eine seiner enantiomeren Formen oder in Form eines Gemisches der letzteren, worin R dieselbe Bedeutung wie in Formel (I) hat und $R'_1$ eine $C_1$-$C_4$ Alkoxy-Gruppe bedeutet, in Gegenwart von Palladium auf Kohle, was zu Verbindungen der Formel

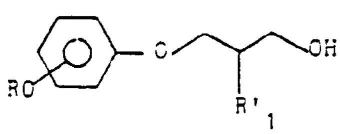

(Id)

führt, worin R und $R'_1$ dieselben Bedeutungen wie in Formel (XII) haben;

(e) der Alkylierung der Verbindungen (Id) in Gegenwart eines Metallhydrids durch eine Verbindung der Formel

$R_4$ - Z (XI)

worin Z eine Abgangsgruppe ist und $R_4$ eine $C_1$-$C_4$ Alkyl-Gruppe bedeutet, was zu Verbindungen der Formel

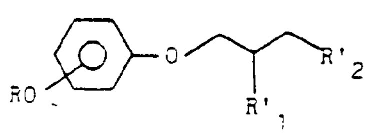

(Ie)

führt, worin R und $R'_1$ dieselben Bedeutungen wie in Formel (XII) haben und $R'_2$ = $C_1$-$C_4$ Alkoxy;

(f) der Reaktion mit den Verbindungen der Formel

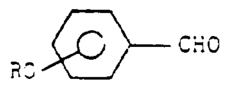

(XIV)

worin R dieselbe Bedeutung hat wie in Formel (I), mit einer Organomagnesiumverbindung der Formel

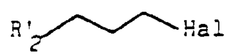

(XV)

worin $R'_2$ eine $C_1$-$C_4$ Alkoxy-Gruppe und Hal ein Halogen-Atom bedeuten, was zu einem Gemisch der Verbindungen der Formeln

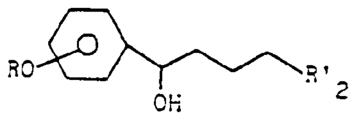

(If)

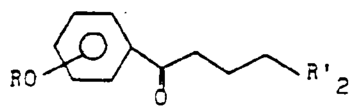

(Ig)

führt, worin R dieselbe Bedeutung wie in Formel (I) hat, und $R'_2$ eine $C_1$-$C_4$ Alkoxy-Gruppe bedeutet, und diese Alkylierung von der Trennung der Verbindungen (If) und (Ig) durch Chromatographie gefolgt wird;

(g) der Alkylierung der Verbindungen der Formel

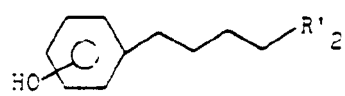

(XVI)

worin $R'_2$ eine $C_1$-$C_4$ Alkoxy-Gruppe bedeutet, durch die Verbindungen (IV) in Gegenwart einer Base, was zu den Verbindungen der Formel

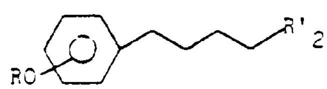

(Ih)

führt, worin R und $R'_2$ dieselben Bedeutungen wie in Formel (If) haben;

(h) der Alkylierung von Phenolen der Formel

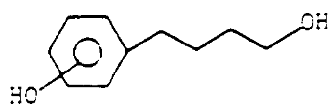

(XVIII)

durch die Verbindungen der Formel (IV) in Gegenwart einer Base, was zu den Verbindungen der Formel

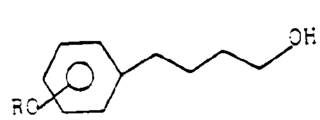

(Ii)

führt, worin R dieselbe Bedeutung wie in Formel (I) hat;

(i) der Alkylierung von Phenolen der Formel

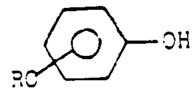

(XX)

worin R dieselbe Bedeutung wie in Formel (I) hat, durch die Verbindungen der Formel

$$Z\text{-}CH_2CH_2CH_2\text{-}OH \qquad (XXI)$$

worin Z eine Abgangsgruppe ist, in Gegenwart einer Base, was zu den Verbindungen der Formel

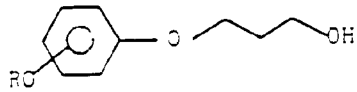

(Ij)

führt, worin R dieselbe Bedeutung wie in Formel (I) hat, gefolgt von einer O-Alkylierung letzterer durch die Verbindungen der Formel (XI) in Gegenwart eines Metallhydrids, was zu den Verbindungen der Formel

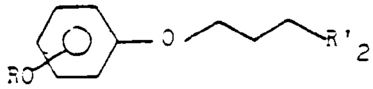

(Ik)

führt, worin R dieselbe Bedeutung wie in Formel (I) hat, und $R'_2$ eine $C_1$-$C_4$ Alkoxy-Gruppe bedeutet;
(j) die Reaktion von Natriumcyanid mit den Verbindungen der Formel

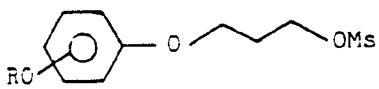

(XXII)

oder

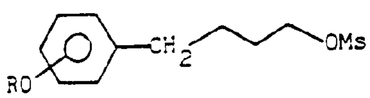

(XXIV)

worin R dieselbe Bedeutung wie in Formel (I) hat, und Ms eine Mesyl-Gruppe bedeutet, was zu den Verbindungen der Formel

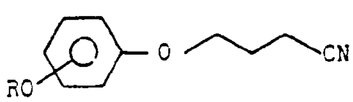

(Il)

beziehungsweise

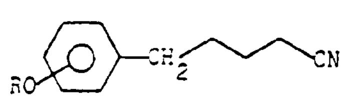

(In)

führt, worin R dieselbe Bedeutung wie in Formel (I) hat;
(k) der Reaktion von N-Alkylaminen der Formel

$$H_2N\text{-}R_4 \qquad (XXIII)$$

worin $R_4$ eine $C_1$-$C_4$ Alkyl-Gruppe bedeutet, mit den Verbindungen der Formel (XXII) oder (XXIV), was zu den Verbindungen der Formeln

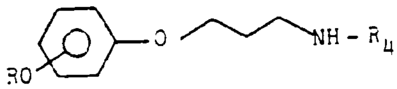

(Im)

oder

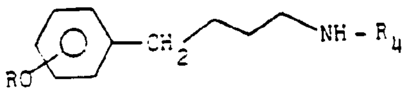

(Io)

führt, worin R dieselbe Bedeutung wie in Formel (I) hat, und $R_4$ eine $C_1$-$C_4$ Alkyl-Gruppe bedeutet;
(l) die saure Hydrolyse der Verbindungen der Formel

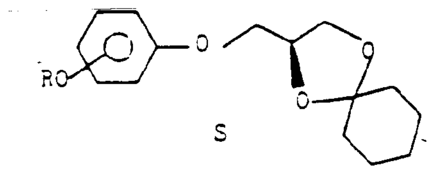

(XXVI)

oder

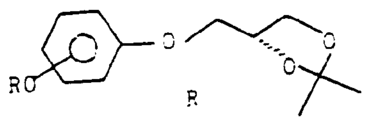

(XXVIII)

worin R dieselbe Bedeutung wie in Formel (I) hat, was zu den Verbindungen der Formel

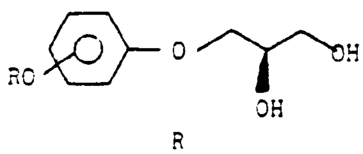

(I'a)

oder

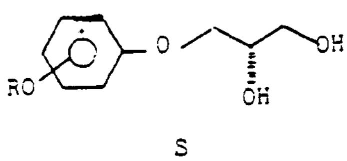

(I''a);

und
führt; und
(m) der Einwirkung von Tosylchlorid auf die Verbindungen der Formel (I'a) oder (I''a), gefolgt von der Reaktion der daraus folgenden Tosylate mit einem dem Alkohol der Formel $R'_2H$, worin $R'_2$ eine $C_1$-$C_4$ Alkyl-Gruppe bedeutet, entsprechenden Alkalimetallalkoholat, was zu den Verbindungen der Formel

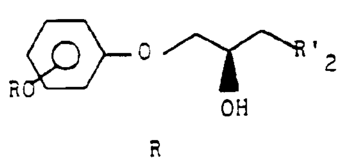

(I'c)

oder

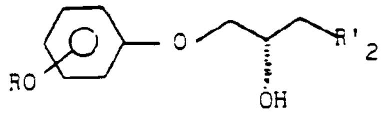

(I''c)

S

führt, umfaßt.

**Patentansprüche für folgende Vertragsstaaten: ES, GR**

1. Verfahren zur Herstellung von Verbindungen der Formel

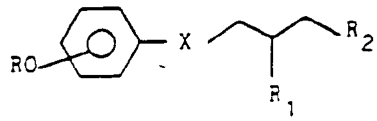

(I)

in welcher

– R eine $C_3$-$C_6$ Alkyl-Gruppe oder eine Benzyl-Gruppe der Formel

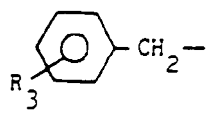

bedeutet, worin $R_3$ = H, Halogen, $C_1$-$C_4$ Alkyl, $C_1$-$C_4$ Alkoxy, $CF_3$, $NO_2$ oder CN; und

– $R_2$

. eine $C_1$-$C_4$ Alkoxy-Gruppe bedeutet, in welchem Fall die Paarung (X, $R_1$) = (O, OH), (O, $C_1$-$C_4$ Alkoxy), (CHOH, H), (O, H), ($CH_2$, H) oder (CO, H);

. eine OH-Gruppe bedeutet, in welchem Fall die Paarung (X, $R_1$) = (O, OH), (O, $C_1$-$C_4$ Alkoxy), (O, H) oder ($CH_2$, H), mit den Maßgaben, daß:

* wenn (X, $R_1$) = (O, OH), R von $C_3$-$C_6$ Alkyl und Benzyl verschieden ist, und

* wenn (X, $R_1$) = (O, H), RO von para -( Cl 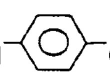 $CH_2O$ ) und o-Benzyloxy verschieden ist;

. eine CN-Gruppe bedeutet, in welchem Fall die Paarung (X, $R_1$) = (O, H) oder ($CH_2$, H); oder

. eine NH-$C_1$-$C_4$ Alkyl-Gruppe bedeutet, in welchem Fall die Paarung (X, $R_1$) = (O, H) oder ($CH_2$, H), mit der Maßgabe, daß, wenn (X, $R_1$) = (O, H) und $R_2$ = NH-$CH_3$ oder

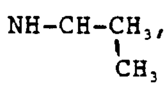

RO von p-Benzyloxy verschieden ist,

dadurch gekennzeichnet, daß es

(a) die Alkylierung von Phenolen der Formel

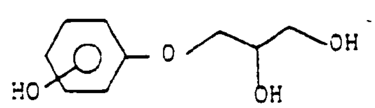

(III)

durch Verbindungen der Formel

Ra - Z (IVa)

worin Z eine Abgangsgruppe ist und Ra dieselbe Bedeutung wie R in der Formel (I) mit Ausnahme von $C_3$-$C_6$ Alkyl und Benzyl hat, was zu Verbindungen der Formel

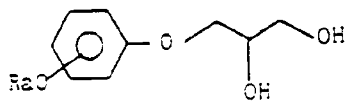

(Ia)

führt;
(b) der Kondensation von Verbindungen der Formel

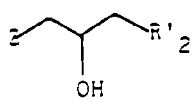

(VI)

worin Z eine Abgangsgruppe ist und $R'_2$ eine $C_1$-$C_4$ Alkoxy-Gruppe beziehungsweise ein Benzyloxyphenol ist, in Gegenwart einer Base und eines Phasentransferkatalysators in einem Zweiphasensystem, was zu Verbindungen der Formel

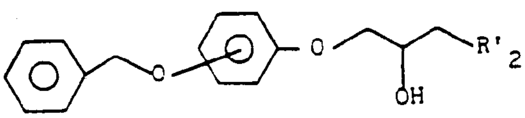

(Ib)

führt, worin $R'_2$ dieselbe Bedeutung wie in Formel (VI) hat;
(c) die Alkylierung von Verbindungen der Formel

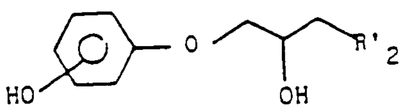

(VII)

worin $R'_2$ dieselbe Bedeutung wie in Formel (VI) hat, durch die Verbindungen der Formel

R - Z (IV)

worin R dieselbe Bedeutung wie in Formel (I) hat und Z eine Abgangsgruppe ist, was zu Verbindungen der Formel

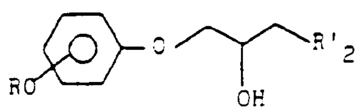

(Ic)

führt, worin R dieselbe Bedeutung wie in Formel (I) hat und $R'_2$ dieselbe Bedeutung wie in Formel (VI) hat;
(d) die Spaltung von Verbindungen der Formel

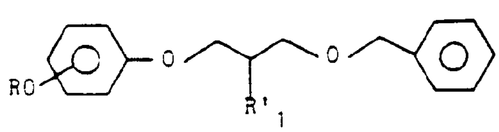

(XII)

durch Hydrierung in eine seiner enantiomeren Formen oder in Form eines Gemisches der letzteren, worin R dieselbe Bedeutung wie in Formel (I) hat und $R'_1$ eine $C_1$-$C_4$ Alkoxy-Gruppe bedeutet, in Gegenwart von Palladium auf Kohle, was zu Verbindungen der Formel

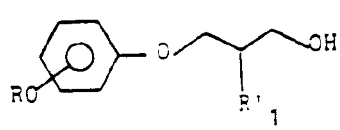

(Id)

führt, worin R und $R'_1$ dieselben Bedeutungen wie in Formel (XII) haben:
(e) der Alkylierung der Verbindungen (Id) in Gegenwart eines Metallhydrids durch eine Verbindung der Formel

$$R_4 - Z \qquad (XI)$$

worin Z eine Abgangsgruppe ist und $R_4$ eine $C_1$-$C_4$ Alkyl-Gruppe bedeutet, was zu Verbindungen der Formel

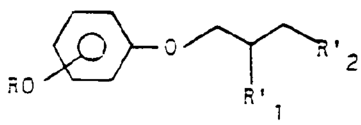

(Ie)

führt, worin R und $R'_1$ dieselben Bedeutungen wie in Formel (XII) haben und $R'_2$ = $C_1$-$C_4$ Alkoxy;
(f) der Reaktion mit den Verbindungen der Formel

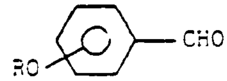

(XIV)

worin R dieselbe Bedeutung hat wie in Formel (I), mit einer Organoagnesiumverbindung der Formel

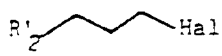

(XV)

worin $R'_2$ eine $C_1$-$C_4$ Alkoxy-Gruppe und Hal ein Halogen-Atom bedeuten, was zu einem Gemisch der Verbindungen der Formeln

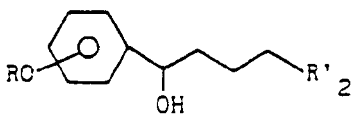

(If)

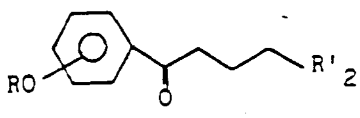

(Ig)

führt, worin R dieselbe Bedeutung wie in Formel (I) hat, und $R'_2$ eine $C_1$-$C_4$ Alkoxy-Gruppe bedeutet, und diese Alkylierung von der Trennung der Verbindungen (If) und (Ig) durch Chromatographie gefolgt wird;
(g) der Alkylierung der Verbindungen der Formel

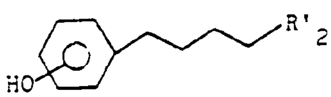

(XVI)

worin $R'_2$ eine $C_1$-$C_4$ Alkoxy-Gruppe bedeutet, durch die Verbindungen (IV) in Gegenwart einer Base, was zu den Verbindungen der Formel

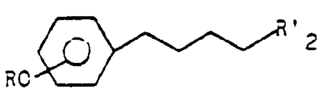

(Ih)

führt, worin R und $R'_2$ dieselben Bedeutungen wie in Formel (If) haben;
(h) der Alkylierung von Phenolen der Formel

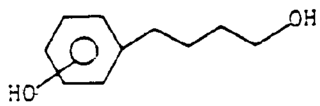

(XVIII)

durch die Verbindungen der Formel (IV) in Gegenwart einer Base, was zu den Verbindungen der Formel

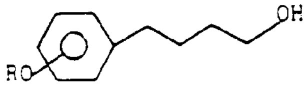

(Ii)

führt, worin R dieselbe Bedeutung wie in Formel (I) hat;
(i) der Alkylierung von Phenolen der Formel

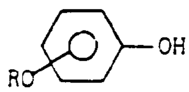

(XX)

worin R dieselbe Bedeutung wie in Formel (I) hat, durch die Verbindungen der Formel

$Z\text{-}CH_2CH_2CH_2\text{-}OH$ (XXI)

worin Z eine Abgangsgruppe ist, in Gegenwart einer Base, was zu den Verbindungen der Formel

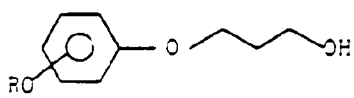

(Ij)

führt, worin R dieselbe Bedeutung wie in Formel (I) hat, gefolgt von einer O-Alkylierung letzterer durch die Verbindungen der Formel (XI) in Gegenwart eines Metallhydrids, was zu den Verbindungen der Formel

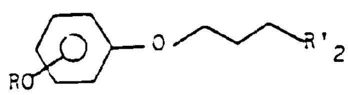

(Ik)

führt, worin R dieselbe Bedeutung wie in Formel (I) hat, und $R'_2$ eine $C_1$-$C_4$ Alkoxy-Gruppe bedeutet;
(j) die Reaktion von Natriumcyanid mit den Verbindungen der Formel

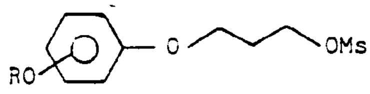

(XXII)

oder

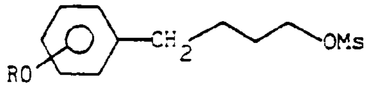

(XXIV)

worin R dieselbe Bedeutung wie in Formel (I) hat, und Ms eine Mesyl-Gruppe bedeutet, was zu den Verbindungen der Formel

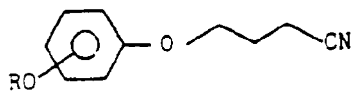
(Il)

beziehungsweise

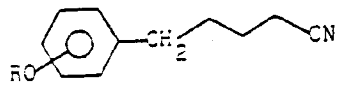
(In)

führt, worin R dieselbe Bedeutung wie in Formel (I) hat;
(k) der Reaktion von N-Alkylaminen der Formel

$H_2N-R_4$ (XXIII)

worin $R_4$ eine $C_1$-$C_4$ Alkyl-Gruppe bedeutet, mit den Verbindungen der Formel (XXII) oder (XXIV), was zu den Verbindungen der Formel

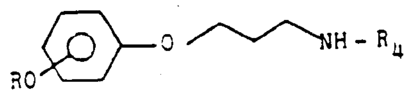
(Im)

oder

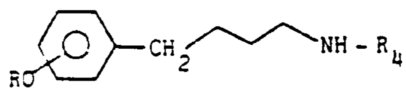
(Io)

führt, worin R dieselbe Bedeutung wie in Formel (I) hat, und $R_4$ eine $C_1$-$C_4$ Alkyl-Gruppe bedeutet;
(l) die saure Hydrolyse der Verbindungen der Formel

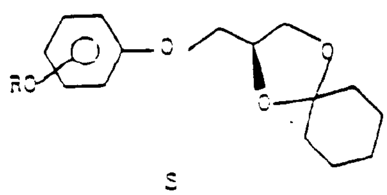
(XXVI)

oder

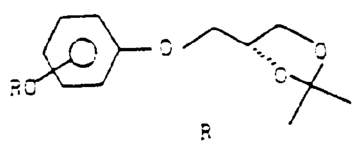
(XXVIII)

worin R dieselbe Bedeutung wie in Formel (I) hat, was zu den Verbindungen der Formel

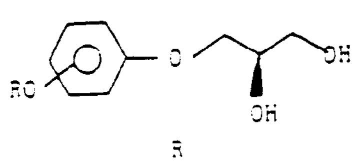
(I'a)

oder

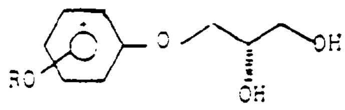

(I''a)

S

führt; und

(m) der Einwirkung von Tosylchlorid auf die Verbindungen der Formel (I'a) oder (I''a), gefolgt von der Reaktion der daraus folgenden Tosylate mit einem dem Alkohol der Formel $R'_2H$, worin $R'_2$ eine $C_1$-$C_4$ Alkyl-Gruppe bedeutet, entsprechenden Alkalimetallalkoholat, was zu den Verbindungen der Formel

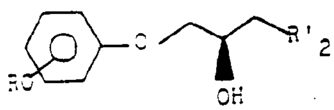

(I'c)

R

oder

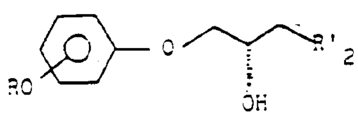

(I''c)

S

führt, umfaßt.

2. Verfahren gemäß Anspruch 1, dadurch gekennzeichnet, daß die Alkylierung (a), (c), (g), (h) oder (i) in Gegenwart von $K_2CO_3$ durchgeführt wird.

## Claims

### Claims for the following Contracting States: AT, DE, BE, IT, LU, NL, GB, SE, CH, LI

1. Compounds with the formula:

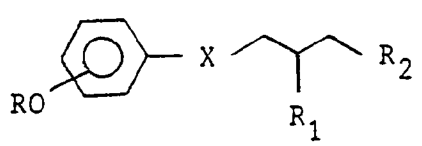

(I)

where :

– R represents a $C_3$-$C_6$ alkyl group or a benzyl group with the formula:

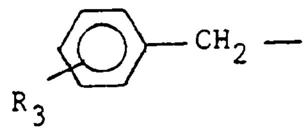

in which $R_3$ = H, a halogen, a $C_1$-$C_4$ alkyl, a $C_1$-$C_4$ alkoxy, $CF_3$, $NO_2$ or a CN group; and

– $R_2$ represents:

. a $C_1$-$C_4$ alkoxy group, in which case the pair (X, $R_1$) (O, OH), (O, $C_1$-$C_4$ alkoxy, (CHOH, H), (O, H), ($CH_2$, H) or (CO, H);

. an OH group, in which case the pair (X, $R_1$) = (O, OH), (O, $C_1$-$C_4$ alkoxy), (O, H) or ($CH_2$, H), with the reservations that:

* when (X, $R_1$) = (O, OH), R is different from a $C_3$-$C_6$ alkyl or a benzyl group; and

* when (X, $R_1$)= (O, H), RO is different from para-(Cl-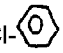 -$CH_2O$) and ortho-benzyloxy ;

. a CN group, in which case the pair (X, $R_1$) = (O, H) or ($CH_2$, H); or

. a $C_l$ -$C_4$ alkyl-NH group, in which case the pair (X, $R_l$) = (O,H) or ($CH_2$,H) with the reservation that when (X, $R_l$) = (O,H) and $R_2$ = NH-$CH_3$ or

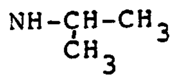 ,

RO is different from para-benzyloxy.

2. Compounds as in Claim I, in which RO is situated in the para position.

3. Compounds as in Claim 2, in which $R_3$ is situated in the meta position.

4. Compounds as in any of the preceding Claims, in which $R_1$ is a methoxy group.

5. Compounds as in any of Claims 1 to 4, in which $R_2$ is a methoxy group.

6. A pharmaceutical or veterinary preparation containing (a) at least one compound with the formula:

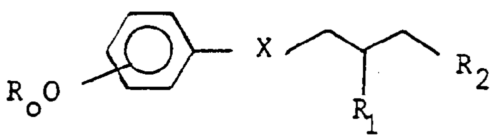

(Io)

where:

– $R_o$ represents a $C_3$-$C_6$ alkyl group or a benzyl group with the formula:

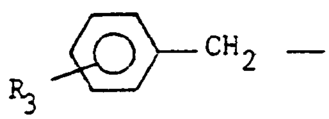

in which $R_3$ = H, a halogen, a $C_1$-$C_4$ alkyl, a $C_1$-$C_4$ alkoxy, $CF_3$, $NO_2$ or a CN group; and

– $R_2$ represents:

. a $C_1$-$C_4$ alkoxy group, in which case the pair (X, $R_1$) = (O, OH), (O, $C_1$-$C_4$ alkoxy), (CHOH, H), (O, H), ($CH_2$ , H) or (CO, H);

. an OH group, in which case the pair (X, $R_1$) = (O, OH), (O, $C_1$-$C_4$ alkoxy), (O, H) or ($CH_2$, H), with the reservation that when (X, $R_1$) = (O, OH), $R_o$ is different from a $C_3$-$C_6$ alkyl or a benzyl group; or

. a CN or $C_1$-$C_4$ alkyl-NH group, in which case the pair (X, $R_1$) = (O, H) or ($CH_2$, H),

together with (b) a carrier or vehicle which is physiologically acceptable and appropriate for the compound used.

7. The use of the compounds (Io) as specified in Claim 6 for the preparation of a selective inhibitor for type B monoamine oxydase.

8. A process for the preparation of compounds with the formula:

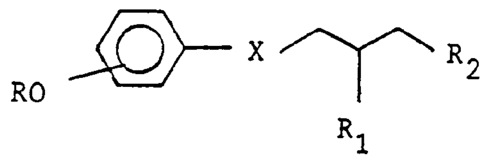

(I)

where:

– R represents a $C_3$-$C_6$ alkyl group or a benzyl group with the formula:

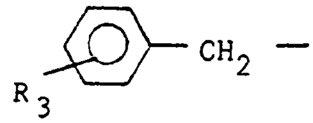

in which $R_3$ = H, a halogen, a $C_1$-$C_4$ alkyl, a $C_1$-$C_4$ alkoxy, $CF_3$, $NO_2$ or a CN group; and

– $R_2$ represents:

. a $C_1$-$C_4$ alkoxy group, in which case the pair (X, $R_1$) = (O, OH), (O, $C_1$-$C_4$ alkoxy), (CHOH, H), (O, H), ($CH_2$, H) or (CO, H);

. an OH group, in which case the pair (X, $R_1$) = (O, OH), (C, $C_1$-$C_4$ alkoxy), (O, H) or ($CH_2$, H), with the reservations that:

* when (X, $R_1$) = (O, OH), R is different from a $C_3$-$C_6$ alkyl or a benzyl group; and

* when (X, $R_1$) = (O, H) RO is different from para -(Cl 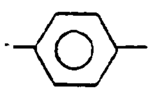 $CH_2O$) and ortho-benzyloxy;

. a CN group, in which case the pair (X, $R_1$) = (O, H) or ($CH_2$, H); or

. a $C_l$-$C_4$ alkyl-NH group, in which case the pair (X, $R_l$) = (O,H) or ($CH_2$,H) with the reservation that when (X, $R_l$) = (O, H) and $R_2$ = NH-$CH_3$ or

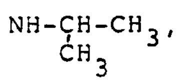

RO is different from para-benzyloxy,

characterized in that it comprises:

(a) the alkylation of phenols with the formula:

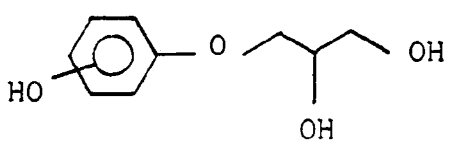

(III)

respectively by means of compounds with the formula:

Ra - Z (IVa)

where Z is a readily substitutable group and Ra has the same signification as R in formula (I), with the exception of $C_3$-$C_6$ alkyl and benzyl, thereby producing compounds with the formula:

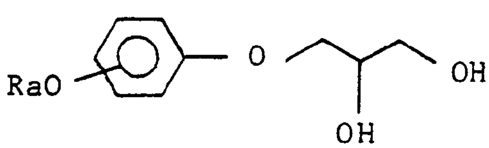

(Ia)

(b) the condensation of compounds with the formula:

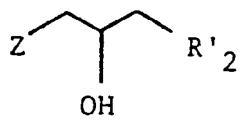

(VI)

where Z is a readily substitutable group and $R'_2$ is a $C_1$-$C_4$ alkoxy group, respectively on a benzyloxyphenol, in the presence of a base and a phase transfer catalyst in a two-phase medium, thereby producing compounds with the formula:

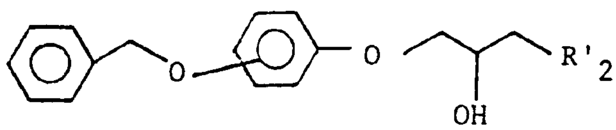

(Ib)

where $R'_2$ has the same signification as in formula (VI);
(c) the alkylation of compounds with the formula:

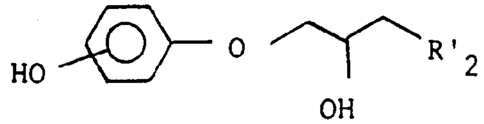

(VII)

where R'2 has the same signification as in formula (VI), respectively by means of compounds with the formula:

R - Z (IV)

where R has the same signification as in formula (I) and Z is a readily substitutable group, thereby producing compounds with the formula:

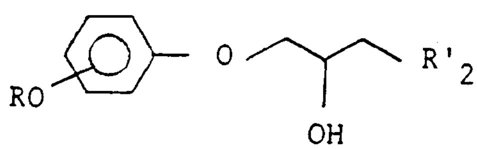

(Ic)

where R has the same signification as in formula (I) and R'2 has the same signification as in formula (VI);
(d) the cleavage by hydrogenation of compounds with the formula:

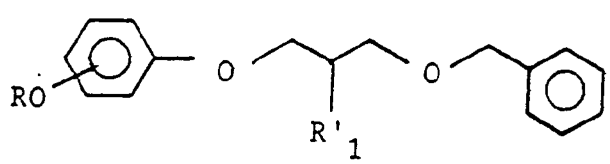

(XII)

in the form of one of their enantiomers or a mixture thereof,
where R has the same signification as in formula (I) and $R'_1$ represents a $C_1$-$C_4$ alkoxy group, in the presence of palladium on carbon, thereby producing compounds with the formula:

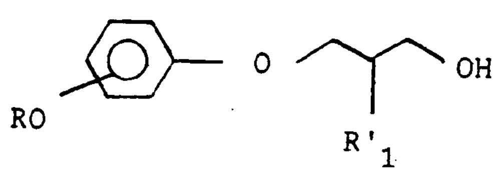

(Id)

where R and $R'_1$ have the same significations as in formula (XII);

(e) the alkylation of compounds (Id) in the presence of a metal hydride, respectively by means of a compound with the formula:

$R_4$ - Z (XI)

where Z is a readily substitutable group and $R_4$ represents a $C_1$-$C_4$ alkyl group, thereby producing compounds with the formula:

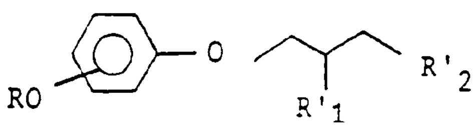

(Ie)

where R and $R'_1$ have the same significations as in formula (XII) and $R'_2$ is a $C_1$-$C_4$ alkoxy group;
(f) the reaction of compounds with the formula:

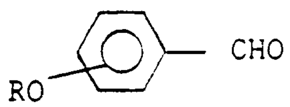

(XIV)

where R has the same signification as in formula (I), with a Grignard reagent of a compound having the formula:

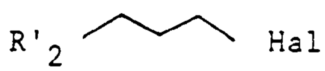

(XV)

where $R'_2$ represents a $C_1$-$C_4$ alkoxy group an Hal represents a halogen atom, thereby producing a mixture of compounds with the formulae:

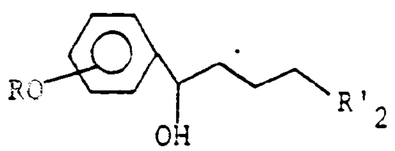

(If)

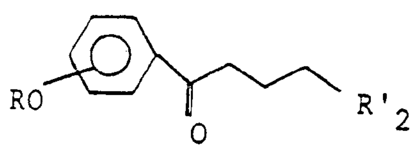

(Ig)

where R has the same signification as in formula (I) and $R'_2$ represents a $C_1$-$C_4$ alkoxy group, the said alkylation reaction being followed by the chromatographic separation of compounds (If) and (Ig);
(g) the alkylation, in the presence of a base, of compounds with the formula:

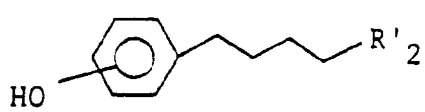

(XVI)

where $R'_2$ represents a $C_1$-$C_4$ alkoxy group, respectively by means of the compounds (IV), thereby producing compounds with the formula:

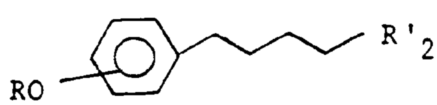

(Ih)

where R and $R'_2$ have the same significations as in formula (If);
(h) the alkylation, in the presence of a base, of phenols with the formula:

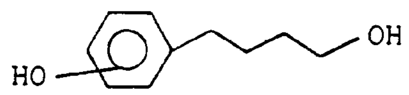

(XVIII)

respectively by means of compounds with the formula (IV), thereby producing compounds with the formula:

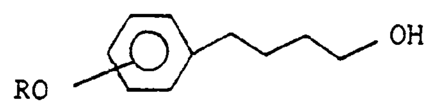

(Ii)

where R has the same signification as in formula (I);
(i) the alkylation, in the presence of a base,of phenols with the formula:

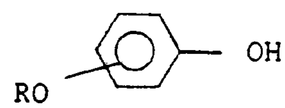

(XX)

where R has the same signification as in formula (I), respectively by means of compounds with the formula:

$$Z - CH_2CH_2CH_2 - OH \qquad (XXI)$$

where Z is a readily substitutable group, thereby producing compounds with the formula:

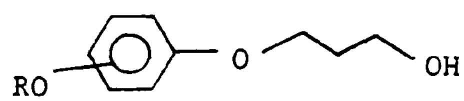

(Ij)

where R has the same signification as in formula (I), followed by O-alkylation of the said compounds by means of compounds with the formula (XI), in the presence of a metal hydride, thereby producing compounds with the formula:

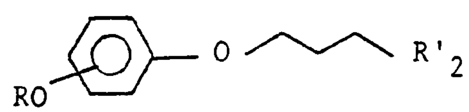

(Ik)

where R has the same signification as in formula (I) and $R'_2$ represents a $C_1$-$C_4$ alkoxy group;
(j) the reaction of sodium cyanide on compounds with the formulae:

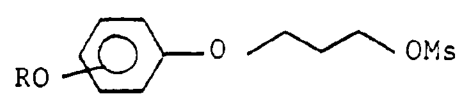

(XXII)

or:

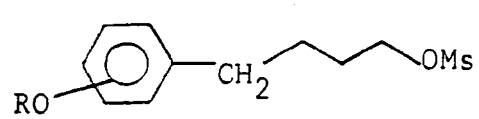

(XXIV)

where R has the same signification as in formula (I) and Ms represents a mesyl group, thereby producing compounds with the formulae:

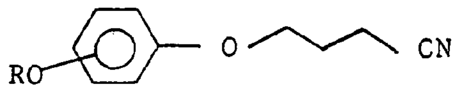

(Il)

or:

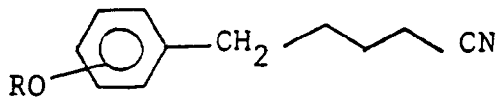

(In)

where R has the same signification as in formula (I);

(k) the reaction of N-alkylamines with the formula:

$H_2N - R_4$ (XXIII)

where $R_4$ represents a $C_1$-$C_4$ alkyl group, on the compounds with formulae (XXII) or (XXIV), thereby producing compounds with the formulae:

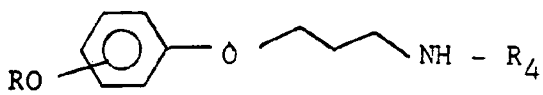

(Im)

or:

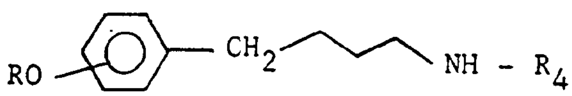

(Io)

where R has the same signification as in formula (I) and $R_4$ represents a $C_1$-$C_4$ alkyl group;

(l) the acidic hydrolysis of compounds with the formulae:

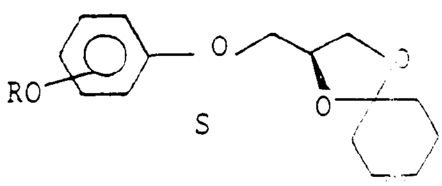

(XXVI)

or:

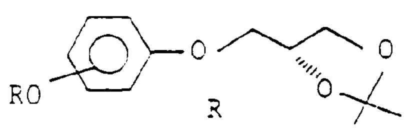

(XXVIII)

where R has the same signification as in formula (I), thereby producing compounds with the formulae:

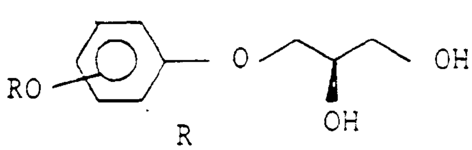

(I'a)

or:

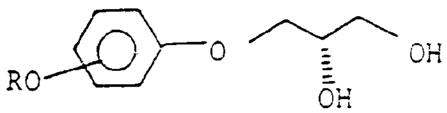

S

(I''a);

and;

(m) the action of tosyl chloride on compounds with the formulae (I'a) or (I''a), followed by the reaction of the resulting tosylates respectively with an alkali metal alcoholate corresponding to the alcohol with the formula $R'_2H$, where $R'_2$ represents a $C_1$-$C_4$ alkoxy group, thereby producing compounds with the formulae:

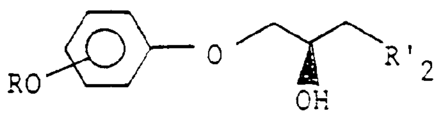

R

(I'c)

or:

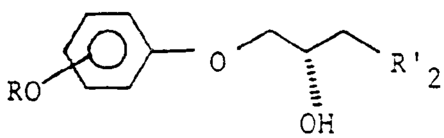

S

(I''c)

**Claims for the following Contracting States: ES, GR**

1. A process for the preparation of compounds with the formula:

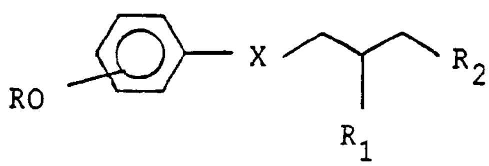

(I)

where:

– R represents a $C_3$-$C_6$ alkyl group or a benzyl group with the formula:

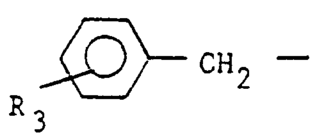

in which $R_3$ = H, a halogen, a $C_1$-$C_4$ alkyl, a $C_1$-$C_4$ alkoxy, $CF_3$, $NO_2$ or a CN group; and

– $R_2$ represents:

. a $C_1$-$C_4$ alkoxy group, in which case the pair (X, $R_1$) = (O, OH), (O, $C_1$-$C_4$ alkoxy), (CHOH, H), (O, H), ($CH_2$, H) or (CO, H);

. an OH group, in which case the pair (X, $R_1$) = (O, OH), (O, $C_1$-$C_4$ alkoxy), (O, H) or ($CH_2$, H), with the reservations that:

* when (X, $R_1$) = (O, OH), R is different from a $C_3$-$C_6$ alkyl or a benzyl group; and

* when (X, $R_1$) = (O, H), RO is different from para -(Cl 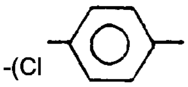 $CH_2O$) and ortho-benzyloxy ;
. a CN group, in which case the pair (X, $R_1$) = (O,H) or ($CH_2$, H); or
. a $C_l$-$C_4$) alkyl-NH group, in which case the pair (X, $R_l$) = (O,H) or ($CH_2$,H) with the reservation that when (X, $R_l$) = (O,H) and $R_2$ = NH-$CH_3$ or

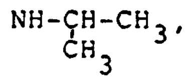

RO is different from para-benzyloxy, characterized in that it comprises :
(a) the alkylation of phenols with the formula:

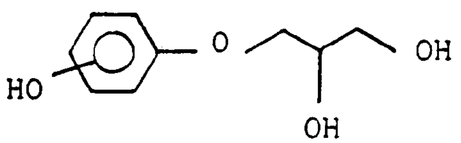
(III)

respectively by means of compounds with the formula:

Ra - Z (IVa)

where Z is a readily substitutable group and Ra has the same signification as R in formula (I), with the exception of $C_3$-$C_6$ alkyl and benzyl, thereby producing compounds with the formula:

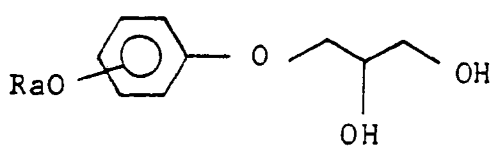
(Ia)

(b) the condensation of compounds with the formula:

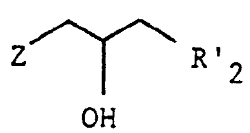
(VI)

where Z is a readily substitutable group and R'$_2$ is a $C_1$-$C_4$ alkoxy group, respectively on a benzyloxyphenol, in the presence of a base and a phase transfer catalyst in a two-phase medium, thereby producing compounds with the formula:

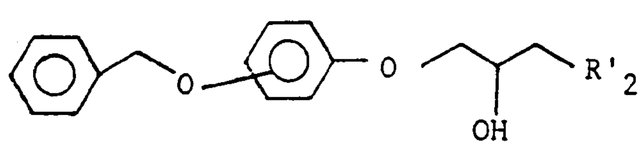
(Ib)

where R'$_2$ has the same signification as in formula (VI);
(c) the alkylation of compounds with the formula:

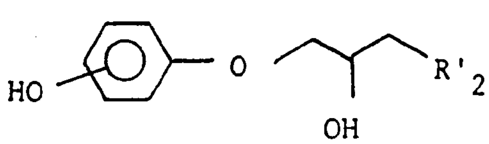
(VII)

where R'2 has the same signification as in formula (VI), respectively by means of compounds with the formula:

$$R - Z \qquad (IV)$$

where R has the same signification as in formula (I) and Z is a readily substitutable group, thereby producing compounds with the formula:

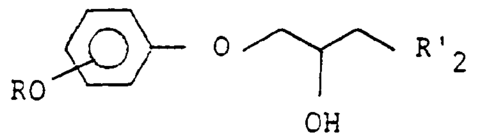

(Ic)

where R has the same signification as in formula (I) and R'2 has the same signification as in formula (VI);
(d) the cleavage by hydrogenation of compounds with the formula:

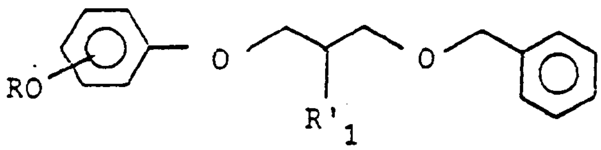

(XII)

in the form of one of their enantiomers or a mixture thereof,
where R has the same signification as in formula (I) and $R'_1$ represents a $C_1$-$C_4$ alkoxy group, in the presence of palladium on carbon, thereby producing compounds with the formula:

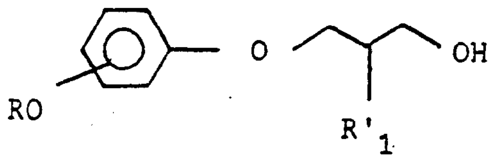

(Id)

where R and $R'_1$ have the same significations as in formula (XII);
(e) the alkylation of compounds (Id) in the presence of a metal hydride, respectively by means of a compound with the formula:

$$R_4 - Z \qquad (XI)$$

where Z is a readily substitutable group and $R_4$ represents a $C_1$-$C_4$ alkyl group, thereby producing compounds with the formula:

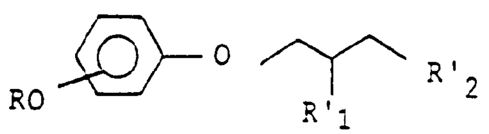

(Ie)

where R and $R'_1$ have the same significations as in formula (XII) and $R'_2$ is a $C_1$-$C_4$ alkoxy group;
(f) the reaction of compounds with the formula:

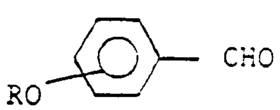

(XIV)

where R has the same signification as in formula (I), with a Grignard reagent of a compound having the formula:

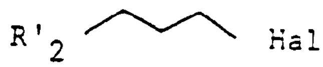

(XV)

where $R'_2$ represents a $C_1$-$C_4$ alkoxy group and Hal represents a halogen atom, thereby producing a mixture of compounds with the formulae:

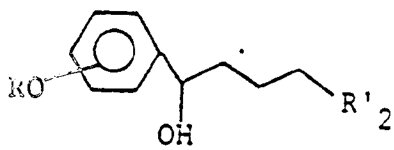

(If)

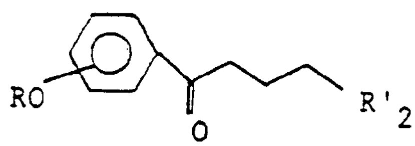

(Ig)

where R has the same signification as in formula (I) and $R'_2$ represents a $C_1$-$C_4$ alkoxy group, the said alkylation reaction being followed by the chromatographic separation of compounds (If) and (Ig);
(g) the alkylation, in the presence of a base, of compounds with the formula:

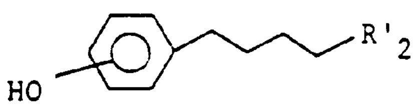

(XVI)

where $R'_2$ represents a $C_1$-$C_4$ alkoxy group, respectively by means of the compounds (IV), thereby producing compounds with the formula:

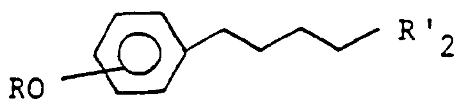

(Ih)

where R and $R'_2$ have the same significations as in formula (If);
(h) the alkylation, in the presence of a base, of phenols with the formula:

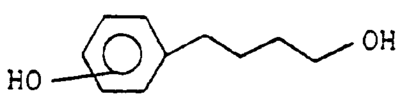

(XVIII)

respectively by means of compounds with the formula (IV), thereby producing compounds with the formula:

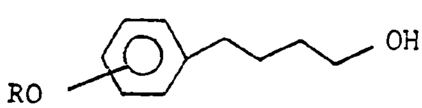

(Ii)

where R has the same signification as in formula (I);
(i) the alkylation, in the presence of a base,of phenols with the formula:

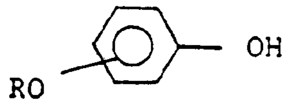

(XX)

where R has the same signification as in formula (I), respectively by means of compounds with the formula:

$$Z - CH_2CH_2CH_2 - OH \qquad (XXI)$$

where Z is a readily substitutable group, thereby producing compounds with the formula:

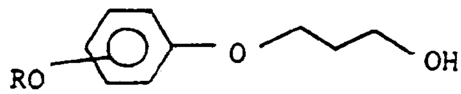

(Ij)

where R has the same signification as in formula (I), followed by O-alkylation of the said compounds by means of compounds with the formula (XI), in the presence of a metal hydride, thereby producing compounds with the formula:

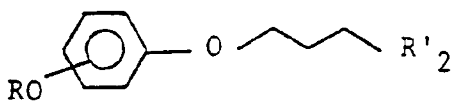

(Ik)

where R has the same signification as in formula (I) and $R'_2$ represents a $C_1$-$C_4$ alkoxy group;
(j) the reaction of sodium cyanide on compounds with the formulae:

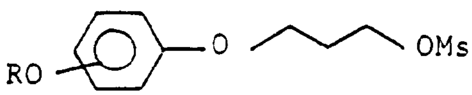

(XXII)

or:

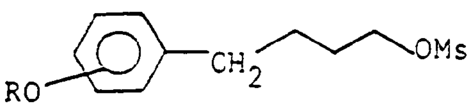

(XXIV)

where R has the same signification as in formula (I) and Ms represents a mesyl group, thereby producing compounds with the formulae:

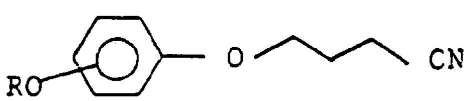

(Il)

or:

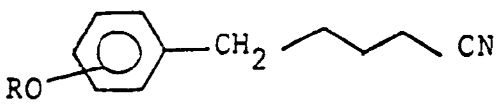

(In)

where R has the same signification as in formula (I);
(k) the reaction of N-alkylamines with the formula:

$$H_2N - R_4 \qquad (XXIII)$$

where $R_4$ represents a $C_1$-$C_4$ alkyl group, on the compounds with formulae (XXII) or (XXIV), thereby producing compounds with the formulae:

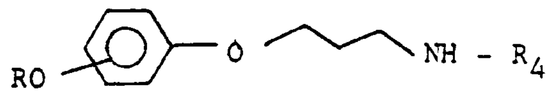

(Im)

or:

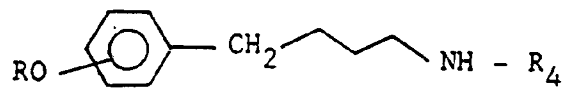

(Io)

where R has the same signification as in formula (I) and $R_4$ represents a $C_1$-$C_4$ alkyl group;
(l) the acidic hydrolysis of compounds with the formulae:

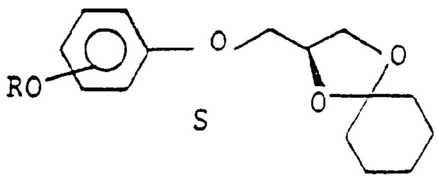

(XXVI)

or:

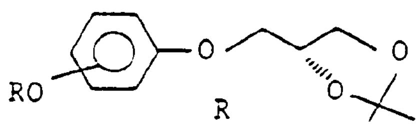

(XXVIII)

where R has the same signification as in formula (I), thereby producing compounds with the formulae:

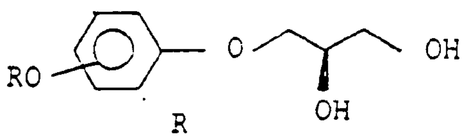

(I'a)

or:

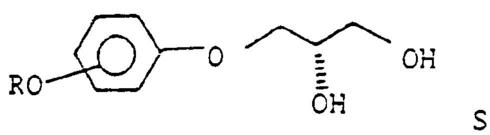

(I"a);

and;
(m) the action of tosyl chloride on compounds with the formulae (I'a) or (I"a), followed by the reaction of the resulting tosylate respectively with an alkali metal alcoholate corresponding to the alcohol with the formula $R'_2H$, where $R'_2$ represents a $C_1$-$C_4$ alkoxy group, thereby producing compounds with the formulae:

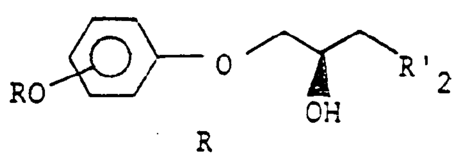

(I'c)

or:

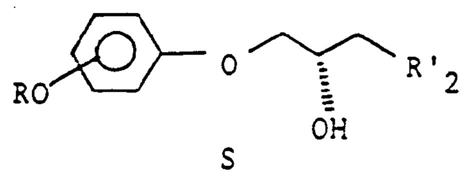

(I"c)

2. Process as in claim I, wherein alkylation (a), (c), (g), (h) or (i) is carried out in the presence of $K_2CO_3$.